(19) Europäisches Patentamt
European Patent Office
Office européen des brevets

(11) EP 1 980 558 A1

(12) # DEMANDE DE BREVET EUROPEEN

(43) Date de publication:
**15.10.2008 Bulletin 2008/42**

(51) Int Cl.:
***C07D 401/06*** *(2006.01)* ***C07D 401/14*** *(2006.01)*
***C07D 403/14*** *(2006.01)* ***C09B 26/04*** *(2006.01)*

(21) Numéro de dépôt: **08154420.7**

(22) Date de dépôt: **11.04.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **13.04.2007 FR 0754454**

(71) Demandeur: **L'Oréal**
**75008 Paris (FR)**

(72) Inventeurs:
- **DAVID, Hervé**
  **94210, LA VARENNE SAINT HILAIRE (FR)**
- **MURGUET, Nadège**
  **91120, PALAISEAU (FR)**

(74) Mandataire: **Wattremez, Catherine**
**L'OREAL**
**River Plaza - DIPI**
**25-29 Quai Aulagnier**
**92665 Asnières-sur-Seine (FR)**

(54) **Composés monochromophoriques polycationiques de type hydrazone, compositions tinctoriales les comprenant et procédé de coloration**

(57) La présente demande a pour objet des composés monochromophoriques polycationiques de type hydrazone particuliers de formule (I) suivante :

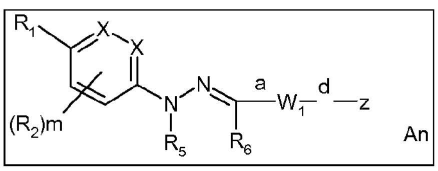

dans laquelle $W_1$ représente un radical hétéroaromatique, à 5 ou 6 chaînons, éventuellement condensé avec un noyau aromatique à 6 chaînons, comprenant un atome d'azote quaternisé et éventuellement un autre hétéroatome identique ou non à l'azote ; X, identiques ou non, représentent un atome d'azote ou un groupement $CR_2$ ; Z représente un groupement cationique comprenant au moins un atome d'azote quaternisé.

La présente invention concerne de même des compositions pour la coloration des fibres kératiniques humaines comprenant de tels composés, un procédé de coloration mettant en jeu une telle composition ainsi que des dispositifs à plusieurs compartiments.

EP 1 980 558 A1

## Description

La présente demande a pour objet des composés monochromophoriques polycationiques de type hydrazone, des compositions pour la coloration des fibres kératiniques humaines comprenant de tels composés. Elle concerne de plus un procédé de coloration mettant en jeu une telle composition ainsi que des dispositifs à plusieurs compartiments.

Il est connu de teindre les fibres kératiniques et en particulier les fibres kératiniques humaines, telles que les cheveux, avec des compositions tinctoriales contenant des colorants directs. Ces composés sont des molécules colorées et colorantes ayant une certaine affinité pour les fibres. Il est connu par exemple d'utiliser des colorants directs de types nitrés benzéniques, anthraquinoniques, nitropyridines, azoïques, xanthéniques, acridiniques, aziniques, triarylméthanes ou encore des colorants directs aromatiques comprenant une fonction hydrazone.

Contrairement aux colorations obtenues en mettant en oeuvre des précurseurs de colorant d'oxydation qui développent la couleur au sein même de la fibre, par un processus de condensation en milieu oxydant alcalin, les colorants directs ne pénètrent pas en profondeur dans la fibre kératinique mais restent localisés plutôt en surface ou dans la fibre proche de la surface.

Ainsi les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur relative mauvaise tenue vis-à-vis de shampooings répétés par exemple. Par ailleurs, ces interactions colorants - fibres peuvent causer des difficultés de mise en oeuvre notamment en ce qui concerne la montée du colorant dans la fibre, ce qui peut aboutir à des colorations insuffisamment homogènes et/ou à des virages de la coloration.

Il a été découvert que l'on pouvait augmenter de manière surprenante l'affinité de colorants monochromophoriques cationiques hydrazone ainsi que leur ténacité vis-à-vis de la lumière ou des shampooings, et cela même sur cheveux sensibilisés, en les substituant par un ou plusieurs groupement cationiques.

Ces buts et d'autres sont atteints par la présente invention qui a donc pour objet des composés monochromophoriques polycationiques de type hydrazone de formule (I) suivante, leurs formes tautomères ainsi que leurs sels d'addition avec un acide et leurs solvates :

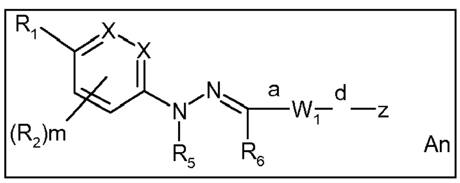
(I)

dans laquelle :

$W_1$ représente un radical hétéroaromatique de formules (1) à (8) suivantes :

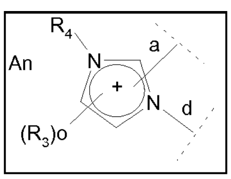
(1)

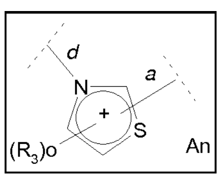
(2)

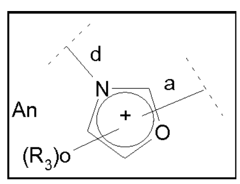
(3)

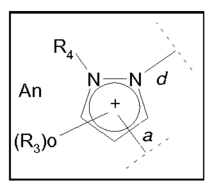
(4)

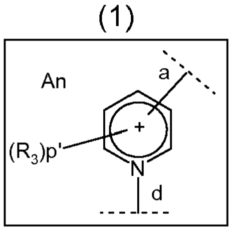
(5)

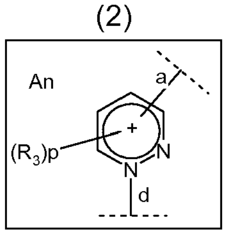
(6)

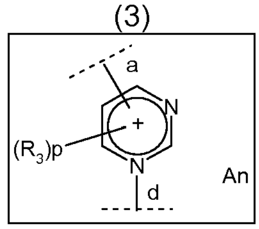
(7)

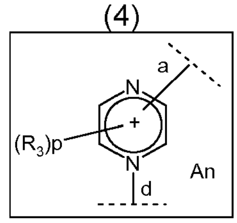
(8)

Où:

* le radical $R_1$ représente :

- un hydrogène ;
- un radical alkyle en $C_1$-$C_{20}$, éventuellement substitué, éventuellement interrompu par un ou plusieurs hétéroatomes et/ou par un ou plusieurs groupements comprenant au moins un hétéroatome, de préférence choisis parmi l'oxygène, l'azote, le soufre, CO, SO, $SO_2$ ou leurs combinaisons ; le radical alkyle ne comportant pas de fonction nitro, nitroso, peroxo ou diazo ;
- un radical alkylcarbonyle (R-CO-) dans lequel R représente un radical alkyle en $C_1$-$C_4$ ;
- un radical alkylsulfonyle ($RSO_2$-) dans lequel R représente un radical alkyle en $C_1$-$C_4$ ;
- un radical arylsulfonyle ($R'SO_2$-) dans lequel R' représente un radical phényle ou benzyle éventuellement substitué ;
- un radical (di-)(alkyl)aminosulfonyle ($(R)_2N$-$SO_2$-) dans lequel les radicaux R indépendamment représentent un hydrogène, un radical alkyle en $C_1$-$C_4$ ;
- un radical (di-)(alkyl) aminocarbonyle $(R)_2N$-CO-) dans lequel les radicaux R indépendamment représentent un hydrogène, un radical alkyle en $C_1$-$C_4$ ;
- un atome d'halogène choisi de préférence parmi le brome, le chlore ou le fluor ;
- un groupement hydroxyle ;
- un groupement alcoxy en $C_1$-$C_4$ ;
- un groupement (poly)hydroxyalcoxy en $C_2$-$C_4$ ;
- un groupement hydroxycarbonyle ;
- un groupement alcoxycarbonyle (RO-CO-) dans lequel R représente un radical alkyle en $C_1$-$C_4$ ;
- un radical alkylcarbonyloxy (RCO-O-) dans lequel R représente un radical alkyle en $C_1$-$C_4$ ;
- un groupement aryloxy éventuellement substitué ;
- un groupement $NR_7R_8$ dans lequel $R_7$ et $R_8$ représentent indépendamment l'un de l'autre :
  - un atome d'hydrogène;
  - un radical alkyle en $C_1$-$C_4$, éventuellement porteur d'au moins un groupement hydroxyle ou alcoxy en $C_1$-$C_2$, lesdits radicaux alkyle pouvant éventuellement former avec l'atome d'azote auquel ils sont rattachés, un hétérocycle comprenant de 5 à 7 chaînons, saturé ou insaturé, éventuellement substitué, éventuellement aromatique, comprenant éventuellement un autre hétéroatome identique ou différent de l'azote ;
  - un radical phényle ; un radical aminophényle ; un radical 4-N,N-diéthylaminophényle ; un radical méthoxyphényle ;
- un groupement alkylcarbonylamino (RCO-NR'-) dans lequel le radical R représente un radical alkyle en $C_1$-$C_4$ et le radical R' représente un atome d'hydrogène ou un radical alkyle en $C_1$-$C_4$ ;
- un groupement uréido ($N(R)_2$-CO-NR'-) dans lequel les radicaux R et R',indépendamment les uns des autres, représentent un atome d'hydrogène, un radical alkyle en $C_1$-$C_4$ ;
- un groupement alkylthio (R-S-) dans lequel le groupement R représente un radical alkyle en $C_1$- $C_4$;
- un groupement alkylsulfonylamino ($RSO_2$-NR'-) dans lequel le radical R', représente un atome d'hydrogène ou un radical alkyle en $C_1$-$C_4$, et le radical R représente un radical alkyle en $C_1$-$C_4$ ;
- un groupement cyano ;
- un groupement trifluorométhyle($CF_3$) ;

* Les radicaux $R_2$ et $R_3$, indépendamment les uns des autres, identiques ou différents représentent :

- un atome d'halogène;
- un radical alkyle en $C_1$-$C_{16}$ éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, $SO_2$ ou leurs combinaisons ; le radical alkyle ne comportant pas de fonction nitro, nitroso, peroxo ou diazo ;
- un radical hydroxyle ;
- un radical alcoxy en $C_1$-$C_4$ ;
- un groupement (poly)hydroxyalcoxy en $C_2$-$C_4$;
- un radical hydroxycarbonyle ;
- un radical alcoxycarbonyle (RO-CO-) dans lequel R représente un radical alkyle en $C_1$-$C_4$;
- un radical alkylcarbonyloxy (RCO-O-) dans lequel R représente un radical alkyle en $C_1$-$C_4$;
- un radical aryloxy éventuellement substitué;
- un radical arylamino éventuellement substitué;
- un radical amino éventuellement substitué par un ou deux radicaux alkyle en $C_1$-$C_4$, identiques ou différents,

éventuellement porteurs d'au moins un groupement hydroxyle ou alcoxy en $C_1$-$C_2$, lesdits radicaux alkyle pouvant éventuellement former avec l'atome d'azote auquel ils sont rattachés, un hétérocycle comprenant de 5 à 7 chaînons, saturé ou insaturé, éventuellement substitué, éventuellement aromatique, comprenant éventuellement un autre hétéroatome identique ou différent de l'azote ;

- un radical alkylcarbonylamino (RCO-NR'-) dans lequel le radical R représente un radical alkyle en $C_1$-$C_4$ et R' représente un hydrogène, un radical alkyle en $C_1$-$C_4$ ;
- un groupement (di-)(alkyl)aminocarbonyle ($(R)_2N$-CO-) dans lequel les radicaux R, indépendamment l'un de l'autre représentent un hydrogène, un radical alkyle en $C_1$-$C_4$ ;
- un radical uréido ($N(R)_2$-CO-NR'-) dans lequel les radicaux R, indépendamment les uns des autres, représentent un radical alkyle en $C_1$-$C_4$ et R' représente un hydrogène, un radical alkyle en $C_1$-$C_4$ ;
- un radical (di-)(alkyl) aminosulfonyle ($(R)_2N$-$SO_2$-) dans lequel le radical R représente un atome d'hydrogène, un radical alkyle en $C_1$-$C_4$ ;
- un radical alkylthio (R-S-) dans lequel le radical R représente un radical alkyle en $C_1$-$C_4$ ;
- un radical alkylsulfonylamino ($RSO_2$-NR'-) dans lequel le radical R représente un radical alkyle en $C_1$-$C_4$, et le radical R' représente un atome d'hydrogène, un radical alkyle en $C_1$-$C_4$ ;
- un radical alkylsulfonyle ($RSO_2$-) dans lequel le radical R représente un radical alkyle en $C_1$-$C_4$ ;
- un radical cyano (-CN) ;
- un radical trifluorométhyle (-$CF_3$) ;

* Deux radicaux $R_2$ adjacents peuvent former entre eux et avec les atomes de carbone auxquels ils sont reliés, un radical (hétéro)cyclique aromatique ou non, à 5 ou 6 chaînons, substitué ou non ;

* L'un des radicaux $R_7$ ou $R_8$ peut former avec l'atome d'azote auquel il est rattaché et avec un radical $R_2$ situé en ortho du groupement $NR_7R_8$, un hétérocycle saturé ou insaturé à 5 ou 6 chaînons substitué ou non

* Les radicaux $R_7$ et $R_8$ peuvent former avec l'atome d'azote auquel ils sont rattachés et chacun avec un radical $R_2$ situé en ortho du groupement $NR_7R_8$, un hétérocycle saturé ou insaturé à 5 ou 6 chaînons substitué ou non

* m est un entier compris entre 0 et 4 lorsque m est inférieur à 4, le ou les atomes de carbone non substitués porte(nt) un atome d'hydrogène ;

* X indépendamment l'un de l'autre représente N, $CR_2$ ;

* o est un entier compris entre 0 et 2 ; lorsque o est inférieur à 2, le ou les atomes de carbone non substitués portent un atome d'hydrogène ;

* Deux radicaux $R_3$ adjacents peuvent former entre eux et avec les atomes de carbone auxquels ils sont rattachés, un cycle aromatique à 6 chaînons, substitué ou non;

* Les radicaux $R_4$, identiques ou différents, représentent :

- un radical alkyle en $C_1$-$C_{20}$, éventuellement substitué, éventuellement interrompu par un ou plusieurs hétéroatomes et/ou par un ou plusieurs groupements comprenant au moins un hétéroatome, de préférence choisis parmi l'oxygène, le soufre, CO, SO, $SO_2$ ou leurs combinaisons ; le radical alkyle ne comportant pas de fonction nitro, nitroso, peroxo ou diazo ;
- un radical triméthylsilylalkyle en $C_1$-$C_4$ ;
- un radical phényle éventuellement substitué ;
- un radical benzyle éventuellement substitué ;

* Le radical $R_5$ représente :

- un hydrogène ;
- un radical alkyle en $C_1$-$C_{20}$, éventuellement substitué, éventuellement interrompu par un ou plusieurs hétéroatomes et/ou par un ou plusieurs groupements comprenant au moins un hétéroatome, de préférence choisis parmi l'oxygène, l'azote, le soufre, CO, SO, $SO_2$ ou leurs combinaisons ; le radical alkyle ne comportant pas de fonction nitro, nitroso, peroxo ou diazo ;
- un radical phényle éventuellement substitué ;
- un radical benzyle éventuellement substitué ;

- un radical alkylcarbonyle (R-CO-) dans lequel R représente un radical alkyle en $C_1$-$C_4$ ;
- un radical alkylsulfonyle ($RSO_2$-) dans lequel R représente un radical alkyle en $C_1$-$C_4$ ;
- un radical arylsulfonyle ($R'SO_2$-) dans lequel R' représente un radical phényle ou benzyle éventuellement substitué ;
- un radical (di-)(alkyl)aminosulfonyle ($(R)_2N$-$SO_2$-) dans lequel les radicaux R indépendamment représentent un hydrogène, un radical alkyle en $C_1$-$C_4$;
- un radical (di-)(alkyl) aminocarbonyle $(R)_2N$-CO-) dans lequel les radicaux R indépendamment représentent un hydrogène, un radical alkyle en $C_1$-$C_4$;

* $R_5$ peut former avec un radical $R_2$ situé en ortho du groupement $NR_5$ et avec l'atome d'azote substitué par $R_5$, un hétérocycle saturé ou insaturé à 5 ou 6 chaînons substitué ou non ;

* Le radical $R_6$, représente :

- un atome d'hydrogène ;
- un radical alkyle en $C_1$-$C_{16}$, éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, $SO_2$, ou leurs combinaisons ; le radical alkyle ne comportant pas de fonction nitro, nitroso, peroxo ou diazo ;
- un radical amino éventuellement substitué par un ou deux radicaux alkyle en $C_1$-$C_4$, identiques ou différents, éventuellement porteurs d'au moins un groupement hydroxyle ou alcoxy en $C_1$-$C_2$, lesdits radicaux alkyle pouvant éventuellement former avec l'atome d'azote auquel ils sont rattachés, un hétérocycle à 5 ou 6 chaînons saturé ou insaturé ou aromatique éventuellement substitué, comprenant éventuellement au moins un autre hétéroatome identique ou différent de l'azote ;
- un radical alkylcarbonylamino (RCO-NR'-) dans lequel le radical R représente un radical alkyle en $C_1$-$C_4$ et R' représente un hydrogène, un radical alkyle en $C_1$-$C_4$ ;
- un radical uréido ($N(R)_2$-CO-NR'-) dans lequel les radicaux R, indépendamment les uns des autres, représentent un radical alkyle en $C_1$-$C_4$ et R' représente un hydrogène, un radical alkyle en $C_1$-$C_4$ ;
- un radical alkylsulfonylamino ($RSO_2$-NR'-) dans lequel le radical R représente un radical alkyle en $C_1$-$C_4$ et R' représente un hydrogène, un radical alkyle en $C_1$-$C_4$ ;
- un radical hydroxycarbonyle (HOOC-) ;
- un radical alcoxycarbonyle en $C_1$-$C_4$ (RO-CO-) ;
- un radical phényle éventuellement substitué ;
- un radical benzyle éventuellement substitué ;

* p est un entier compris entre 0 et 3 ; p' est un entier compris entre 0 et 4 ; lorsque p est inférieur à 3, ou lorsque p' est inférieur à 4, le ou les atomes de carbone non substitués porte(nt) un atome d'hydrogène ;

* La liaison a relie le radical hétéroaromatique $W_1$ à l'atome de carbone du groupement $CR_6$ ;

* Z représente un radical alkylène en $C_2$-$C_{20}$ comprenant au moins un atome d'azote quaternisé ;

* la liaison d relie le(s) groupement(s) cationique(s) Z à un atome d'azote du radical hétéroaromatique $W_1$ ;

* l'électroneutralité des composés de formule (I) étant assurée par un ou plusieurs anions ou mélange d'anions An cosmétiquement acceptables, identiques ou non.

Elle a de même pour objet des compositions pour la coloration de fibres kératiniques humaines, en particulier les cheveux, comprenant un ou plusieurs composés monochromophoriques hydrazone de formule (I) décrite précédemment.

Un autre objet de la présente invention réside dans un procédé de coloration de firers kératiniques humaines consistant à mettre en oeuvre les étapes suivantes :

- on applique ladite composition tinctoriale sur les fibres kératiniques,
- on laisser pauser pendant une durée suffisante pour obtenir l'effet souhaité.

Elle concerne de plus un dispositif à plusieurs compartiments avec un premier compartiment renfermant une composition selon l'invention et un second compartiment renfermant une composition comprenant au moins un agent

oxydant.

Selon une variante de l'invention, le dispositif comprend un premier compartiment renfermant une composition comprenant un ou plusieurs colorants monochromophoriques polycationiques de formule (I), un second compartiment comprenant au moins un précurseur de colorant d'oxydation choisi parmi une ou plusieurs bases d'oxydation éventuellement associées à un ou plusieurs coupleurs, et un troisième compartiment renfermant une composition comprenant au moins un agent oxydant.

On a constaté que les composés à fonction hydrazone définis précédemment, outre les avantages relatifs à leur affinité avec la fibre, permettaient aussi d'élargir à des couleurs pouvant aller jusqu'au violet, la palette de couleur classiquement atteinte avec de tels colorants.
Ils sont également stables en conditions alcalines éclaircissantes.
Par ailleurs ces composés peuvent constituer des intermédiaires de synthèse pour de colorants comprenant plusieurs chromophores reliés entre eux par un ou plusieurs bras de liaison.

Mais d'autres caractéristiques et avantages de la présente invention apparaîtront plus clairement à la lecture de la description et des exemples qui vont être présentés.

Dans ce qui va suivre et à moins qu'une autre indication ne soit donnée, les bornes délimitant un domaine de valeurs sont comprises dans ce domaine.

Au sens de la présente invention, et à moins qu'une indication différente ne soit donnée :

- Un radical alkyle est linéaire ou ramifié,

- Un radical alkyle ou la partie alkyle, d'un radical est dite 'substituée' lorsqu'elle comprend au moins un substituant choisi parmi les groupements :

  - hydroxyle,
  - alcoxy en $C_1$-$C_4$, (poly)hydroxyalcoxy en $C_2$-$C_4$,
  - amino éventuellement substitué par un ou plusieurs groupements alkyle identiques ou différents en $C_1$-$C_4$ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.

- Un radical aryle ou hétéroaryle ou la partie aryle ou hétéroaryle d'un radical est dite 'substituée' lorsqu'elle comprend au moins un substituant porté par un atome de carbone, choisi parmi

  • un radical alkyle en $C_1$-$C_{16}$, de préférence en $C_1$-$C_8$, éventuellement substitué par un ou plusieurs radicaux choisis parmi les radicaux hydroxy, alcoxy en $C_1$-$C_2$, (poly)-hydroxyalcoxy en $C_2$-$C_4$, acylamino, amino substitué par deux radicaux alkyles, identiques ou différents, en $C_1$-$C_4$ , éventuellement porteurs d'au moins un groupement hydroxyle ou, les deux radicaux pouvant former avec l'atome d'azote auquel ils sont rattachés, un hétérocycle à 5 ou 7 chaînons, de préférence de 5 ou 6 chaînons, comprenant éventuellement un autre hétéroatome identique ou différent de l'azote ;
  • un atome d'halogène tel que chlore, fluor ou brome;
  • un groupement hydroxyle
  • un radical alcoxy en $C_1$-$C_2$ ; un radical (poly)-hydroxyalcoxy en $C_2$-$C_4$;
  • un radical amino éventuellement substitué par un ou deux radicaux alkyles, identiques ou différents, en $C_1$-$C_4$ éventuellement porteurs d'au moins un groupement hydroxyle ou amino par deux radicaux alkyles en $C_1$-$C_2$ éventuellement substitués ;
  • un radical alkylcarbonylamino (-NR-COR') dans lequel le radical R est un atome d'hydrogène, un radical alkyle en $C_1$-$C_4$ éventuellement porteur d'au moins un groupement hydroxyle et le radical R' est un radical alkyle en $C_1$-$C_2$ ;
  • un radical (di-)(alkyl-)aminocarbonyl ($(R)_2N$-CO-) dans lequel les radicaux R, identiques ou non, représentent un atome d'hydrogène, un radical alkyle en $C_1$-$C_4$ éventuellement porteur d'au moins un groupement hydroxyle ;
  • un radical hydroxycarbonyle (HOOC-) ;
  • un radical alcoxycarbonyle (RO-CO-) dans lequel le radical R représente un radical alkyle en $C_1$-$C_4$;
  • un radical alkylsulfonylamino ($R'SO_2$-NR-) dans lequel le radical R représente un atome d'hydrogène, un radical alkyle en $C_1$-$C_4$ éventuellement porteur d'au moins un groupement hydroxyle et le radical R' représente un radical alkyle en $C_1$-$C_4$, un radical phényle ;
  • un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxyle.

- La partie cyclique ou hétérocyclique d'un radical non aromatique ou un radical (hétéro-)cyclique est dit substitué lorsqu'il comprend au moins un substituant porté par un atome de carbone choisi parmi les groupements:

  - hydroxyle,
  - alcoxy en $C_1$-$C_4$, (poly)hydroxyalcoxy en $C_2$-$C_4$,
  - alkylcarbonylamino ((RCO-NR'-) dans lequel le radical R' est un atome d'hydrogène, un radical alkyle en $C_1$-$C_4$ éventuellement porteur d'au moins un groupement hydroxyle et le radical R est un radical alkyle en $C_1$-$C_2$, amino substitué par deux groupements alkyle identiques ou différents en $C_1$-$C_4$ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.

Comme indiqué auparavant, un premier objet de l'invention consiste en des composés correspondant à la formule (I) précitée.

Selon une variante de l'invention, les composés polycationiques sont tels que $W_1$ représente un radical hétéroaromatique de formules suivantes :

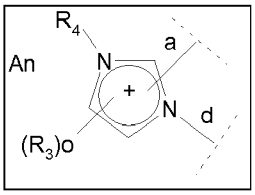

(1)

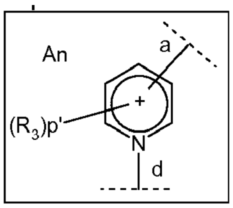

(5)

Les radicaux $R_3$ et $R_4$, o, p', a et d ayant les mêmes significations que précédemment indiqué.

Conformément à un mode de réalisation avantageux de l'invention, les composés polycationiques de formule (I) sont plus particulièrement tels que le radical $R_1$ est choisi parmi :

- un hydrogène ;
- un radical alkyle en $C_1$-$C_4$, éventuellement substitué par un ou plusieurs radicaux identiques ou non choisis parmi les radicaux hydroxy; alcoxy en $C_1$-$C_2$; (poly)hydroxyalcoxy en $C_2$-$C_4$ ; amino substitué par un ou deux alkyle identiques

ou non en $C_1$-$C_2$; thio (-SH) ; thioalkyle en $C_1$-$C_4$ (-RS_); alkyl($C_1$-$C_4$)sulfinyle; alkyl ($C_1$-$C_4$)sulfonyle ;

- un radical alkylcarbonyle (R-CO-) dans lequel R représente un radical alkyle en $C_1$-$C_4$ ;
- un radical alkylsulfonyle ($RSO_2$-) dans lequel R représente un radical alkyle en $C_1$-$C_4$ ; un radical arylsulfonyle ($R'SO_2$-) dans lequel R' représente un radical phényle

ou benzyle éventuellement substitué ;

- un radical (di-)(alkyl)aminosulfonyle ($(R)_2N$-$SO_2$-) dans lequel les radicaux R indépendamment représentent un hydrogène, un radical alkyle en $C_1$-$C_4$ ;
- un radical (di-)(alkyl) aminocarbonyle $(R)_2N$-CO-) dans lequel les radicaux R indépendamment représentent un hydrogène, un radical alkyle en $C_1$-$C_4$ ;
- un atome de chlore
- un groupement hydroxyle ;
- un groupement alcoxy en $C_1$-$C_4$ ;
- un groupement hydroxyalcoxy en $C_2$-$C_4$ ;
- un groupement hydroxycarbonyle ;
- un groupement alcoxycarbonyle (RO-CO-) dans lequel R représente un radical alkyle en $C_1$-$C_4$ ;
- un radical alkylcarbonyloxy (RCO-O-) dans lequel R représente un radical alkyle en $C_1$-$C_4$ ;
- un groupement aryloxy éventuellement substitué ;
- un groupement $NR_7R_8$ dans lequel $R_7$ et $R_8$ représentent indépendamment l'un de l'autre :

- un atome d'hydrogène ;
- un radical alkyle en $C_1$-$C_4$, éventuellement porteur d'au moins un groupement hydroxyle ou alcoxy en $C_1$-$C_2$, lesdits radicaux alkyle pouvant éventuellement former avec l'atome d'azote auquel ils sont rattachés, un hétérocycle comprenant de 5 à 7 chaînons, saturé ou insaturé, éventuellement substitué, éventuellement aromatique, comprenant éventuellement un autre hétéroatome identique ou différent de l'azote ;
- un radical phényle ; un radical aminophényle ; un radical 4-N,N-diéthylaminophényle ; un radical méthoxyphényle ;

- un groupement alkylcarbonylamino (RCO-NR'-) dans lequel le radical R représente un radical alkyle en $C_1$-$C_4$ et le radical R' représente un atome d'hydrogène ou un radical alkyle en $C_1$-$C_4$ ;
- un groupement alkylsulfonylamino ($RSO_2$-NR'-) dans lequel le radical R', représente un atome d'hydrogène ou un radical alkyle en $C_1$-$C_4$, et le radical R représente un radical alkyle en $C_1$-$C_4$.

De préférence, le radical $R_1$ est choisi parmi:

- un hydrogène ;
- un radical alkyle en $C_1$-$C_4$ éventuellement substitué par un groupement hydroxy, alcoxy en $C_1$-$C_2$
- un atome de chlore
- un groupement hydroxyle;
- un groupement alcoxy en $C_1$-$C_4$ ;
- un groupement hydroxyalcoxy en $C_2$-$C_4$ ;
- un groupement hydroxycarbonyle ;
- un groupement alcoxycarbonyle (RO-CO-) dans lequel R représente un radical alkyle en $C_1$-$C_2$ ;
- un groupement aryloxy non substitué ;
- un groupement $NR_7R_8$ dans lequel $R_7$ et $R_8$ représentent indépendamment l'un de l'autre :

  - un atome d'hydrogène ;
  - un radical alkyle en $C_1$-$C_4$, éventuellement porteur d'au moins un groupement hydroxyle ou alcoxy en $C_1$-$C_2$, lesdits radicaux alkyle pouvant éventuellement former avec l'atome d'azote auquel ils sont rattachés, un hétérocycle comprenant de 5 à 7 chaînons, saturé ou insaturé, éventuellement substitué, éventuellement aromatique, comprenant éventuellement un autre hétéroatome identique ou différent de l'azote ;
  - un radical phényle ; un radical 4-N,N-diéthylaminophényle ; un radical méthoxyphényle ;

- un groupement alkylcarbonylamino (RCO-NR'-) dans lequel le radical R représente un radical alkyle en $C_1$-$C_4$ et le radical R' représente un atome d'hydrogène ou un radical alkyle en $C_1$-$C_4$ ;
- un groupement alkylsulfonylamino ($RSO_2$-NR'-) dans lequel le radical R', représente un atome d'hydrogène ou un radical alkyle en $C_1$-$C_2$, et le radical R représente un radical alkyle en $C_1$-$C_2$.

Conformément à un mode de réalisation particulièrement préféré de l'invention, $R_1$ représente un hydrogène, un chlore, un hydroxyle, un méthyle, un éthyle, un radical 2-hydroxyéthyle, un radical 2-méthoxyéthyle, un méthoxy, un groupement hydroxycarbonyle, un groupement méthoxycarbonyle, un groupement éthoxycarbonyle, un groupement amino, (di)méthylamino, (tri)éthylamino, (di)2-hydroxyéthylamino, un groupement pyrrolidine, un groupement phénylamino, 4-methoxyphénylamino, 4-aminophénylamino, 4-N,N-diéthylaminophénylamino, un groupement méthylsulfonylamino ($CH_3$-$SO_2$-NH-), un groupement méthylcarbonylamino ($CH_3CONH$-).

Selon une variante intéressante de l'invention, lorsque $R_1$ représente un radical $-NR_7R_8$, alors l'un des radicaux $R_7$ ou $R_8$ forme avec l'atome d'azote auquel il est rattaché et avec un radical $R_2$ situé en ortho du groupement $NR_7R_8$, un hétérocycle saturé ou insaturé à 5 ou 6 chaînons substitué ou non.

Ainsi, selon cette variante, le groupement $-NR_7R_8$, avec le noyau aromatique éventuellement substitué par un hydroxyle ou méthoxy, peut former l'un des groupements suivants :

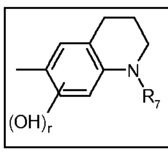
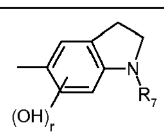
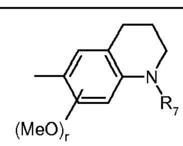
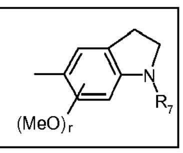

où r vaut 0 ou 1.

Selon une autre variante intéressante de l'invention, lorsque $R_1$ représente un radical $-NR_7R_8$, alors les deux radicaux $R_7$ et $R_8$ forment avec l'atome d'azote auquel ils sont rattachés et chacun avec un radical $R_2$ situé en ortho du groupement $-NR_7R_8$, un hétérocycle saturé ou insaturé à 5 ou 6 chaînons substitué ou non

Par exemple, le groupement $-NR_7R_8$, avec le noyau aromatique éventuellement substitué par un hydroxyle ou un méthoxy, peut former l'un des groupements suivants :

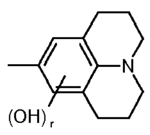 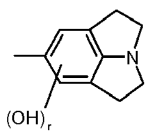 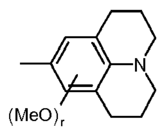 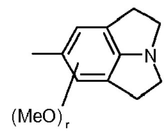 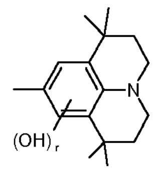

où r vaut 0 ou 1.

En ce qui concerne les radicaux $R_2$, $R_3$, ces derniers, indépendamment les uns des autres, sont choisis plus particulièrement parmi:

- un atome de chlore
- un radical alkyle en $C_1$-$C_{16}$, éventuellement substitué par un ou plusieurs groupements, identiques ou non, choisis parmi les radicaux hydroxy, alcoxy en $C_1$-$C_2$, amino, thio, ((di-)alkyl)amino en $C_1$-$C_2$, alkylsulfonylamino en $C_1$-$C_2$, alkylsulfonyle en $C_1$-$C_2$
- un radical hydroxyle
- un radical alcoxy en $C_1$-$C_4$; un groupement hydroxyalcoxy en $C_2$-$C_4$
- un radical hydroxycarbonyle
- un radical alcoxy($C_1$-$C_4$)carbonyle
- un radical alkyl($C_1$-$C_4$)carbonyloxy
- un radical aryloxy éventuellement substitué
- un radical amino éventuellement substitué par un ou deux radicaux alkyle en $C_1$-$C_4$, identiques ou différents, éventuellement porteurs d'au moins un groupement hydroxyle ou alcoxy en $C_1$-$C_2$, lesdits radicaux alkyle pouvant éventuellement former avec l'atome d'azote auquel ils sont rattachés, un hétérocycle à 5 ou 7 chaînons, saturé ou insaturé, éventuellement substitué, éventuellement aromatique, comprenant éventuellement un autre hétéroatome identique ou différent de l'azote ; par un ou deux radicaux phényle, aminophényle, N,N-diethylaminophényle ou méthoxyphényle
- un radical alkyl($C_1$-$C_4$)carbonylamino
- un radical aminocarbonyle, un groupement (di)alkyl($C_1$-$C_4$)aminocarbonyle
- un radical aminosulfonyle, (di)alkyl($C_1$-$C_4$)aminosulfonyle
- un radical alkyl($C_1$-$C_4$)thio
- un radical alkyl($C_1$-$C_4$)sulfonylamino
- un radical cyano
- un radical phényle
- un radical trifluorométhyle
- un radical thio
- un radical alkyl($C_1$-$C_4$)sulfonyle.

De préférence, les radicaux $R_2$, $R_3$, indépendamment les uns des autres, sont choisis parmi :

- un atome de chlore
- un radical alkyle en $C_1$-$C_8$ éventuellement substitué par un ou plusieurs groupements, identiques ou non, choisis parmi les radicaux hydroxy, alcoxy en $C_1$-$C_2$, amino, thio, ((di-)alkyl)amino en $C_1$-$C_2$, alkylsulfonylamino en $C_1$-$C_2$, alkylsulfonyle en $C_1$-$C_2$
- un radical hydroxyle
- un radical alcoxy en $C_1$-$C_4$ ;
- un radical alcoxy($C_2$-$C_4$)carbonyle
- un radical aryloxy éventuellement substitué
- un radical amino éventuellement substitué :

  • par un ou deux radicaux alkyle en $C_1$-$C_4$, identiques ou différents, éventuellement porteurs d'un groupement

hydroxyle ou alcoxy en $C_1$-$C_2$, lesdits radicaux alkyle pouvant éventuellement former avec l'atome d'azote auquel ils sont rattachés, un hétérocycle à 5 ou 7 chaînons, saturé ou insaturé, éventuellement substitué ;
■ par un ou deux radicaux phényle, aminophényle ou méthoxyphényle

- un radical alkyl($C_1$-$C_4$)carbonylamino dans lequel la fonction amino est substituée ou non par un radical alkyle en $C_1$-$C_4$
- un radical hydroxycarbonyle
- un radical aminocarbonyle
- un radical aminosulfonyle, (di)alkyl($C_1$-$C_4$)aminosulfonyle
- un radical alkyl($C_1$-$C_2$)sulfonylamino
- un radical alkyl($C_1$-$C_2$)sulfonyle
- un radical alkyl($C_1$-$C_4$)thio
- un radical phényle
- un radical trifluorométhyle
- un radical thio.

De préférence, les radicaux $R_2$ et $R_3$, indépendamment les uns des autres représentent un atome de chlore ; un radical méthyle, éthyle, 2-hydroxyéthyle, 2-méthoxyéthyle , un radical hydroxyle; un radical méthoxy, éthoxy ; un radical méthylsulfonyle; un radical méthylcarbonylamino ($CH_3CONH$-); un radical hydroxycarbonyle; un radical amino, (di)méthylamino, (di)éthylamino, (di)2-hydroxyéthylamino ; un radical pyrrolidino ; un radical méthoxycarbonyle, éthoxycarbonyle ; un radical phényle ; un radical méthylsulfonylamino ($CH_3SO_2NH$-) ; un radical 4-méthoxyphénylamino, 4-aminophénylamino.
Conformément à une variante particulière de l'invention, deux radicaux $R_2$ adjacents forment entre eux et avec les atomes de carbone auxquels ils sont reliés, un radical cyclique, éventuellement aromatique, comprenant 5 ou 6 chaînons, substitué ou non. Plus particulièrement, le groupement aromatique formé dans le cadre de cette variante est un noyau phényle non substitué.
Selon une variante avantageuse de l'invention, deux radicaux $R_3$ adjacents forment entre eux et avec les atomes de carbone auxquels ils sont rattachés, un cycle aromatique à 6 chaînons, substitué ou non. Plus particulièrement, le groupement aromatique formé dans le cadre de cette variante est un noyau phényle non substitué.
En ce qui concerne le radical $R_4$, celui-ci représente plus particulièrement :

- un radical alkyle en $C_1$-$C_8$, de préférence en $C_1$-$C_8$, éventuellement substitué par un radical choisi parmi hydroxy, alcoxy en $C_1$-$C_4$, amino éventuellement substitué par un ou deux radicaux alkyle en $C_1$-$C_2$
- un radical triméthylsilylalkyle en $C_1$-$C_4$
- un radical phényle éventuellement substitué par un ou deux groupements choisis parmi le chlore, un radical hydroxy, un radical amino,
- un radical benzyle éventuellement substitué par un ou deux groupements choisis parmi le chlore, un radical hydroxy, un radical amino.

De préférence, le radical $R_4$ représente un radical méthyle, éthyle, propyle, butyle, pentyle, hexyle, heptyle, octyle, 2-hydroxyéthyle, 3-méthoxypropyle, 4-chlorophényle, phényle, benzyle, 3,4-dihydroxybenzyle.
Concernant le radical $R_5$, celui-ci représente plus particulièrement:

- un hydrogène
- un radical alkyle en $C_1$-$C_6$, éventuellement substitué par au moins un groupement hydroxy, par au moins un groupement alcoxy en $C_1$-$C_2$, par au moins un groupement un groupement hydroxycarbonyle
- un radical phényle éventuellement substitué par au moins un atome d'halogène tels que le de chlore, le brome, l'iode, le de fluor ; par au moins un groupement hydroxyle et/ou amino
- un radical benzyle éventuellement substitué par un ou plusieurs hydroxy et/ou amino
- un radical alkyl($C_1$-$C_4$)carbonyle
- un radical alkyl($C_1$-$C_4$)sulfonyle.

Selon un mode de réalisation particulièrement préféré, le radical $R_5$ représente un atome d'hydrogène, un radical méthyle, éthyle, 2-hydroxyéthyle, 2-méthoxyéthyle, acétyle ($CH_3CO$-), méthylsulfonyle ($CH_3SO_2$-), phényle, benzyle éventuellement substitué par un ou plusieurs hydroxy, amino ou leur combinaison.
Conformément à une variante encore plus particulière de l'invention, $R_5$ représente un atome d'hydrogène ; un radical méthyle ; un radical 2-hydroxyéthyle; un radical $CH_3CO$-, un radical $CH_3SO_2$-; un radical benzyle non substitué.
$R_5$ peut également former avec un radical $R_2$ situé en ortho du groupement $R_5$ et avec l'atome d'azote substitué

par $R_5$, un hétérocycle saturé ou insaturé, à 5 ou 6 chaînons, substitué ou non, de préférence non substitué ou substitué par un radical méthyle.

Dans la formule (I), le radical $R_6$ représente de préférence:

- un atome d'hydrogène ;
- un radical alkyle en $C_1$-$C_4$ éventuellement substitué par un ou plusieurs radicaux choisis parmi les radicaux hydroxy, alcoxy en $C_1$-$C_2$, (poly)-hydroxyalcoxy en $C_2$-$C_4$, amino, (di)alkylamino en $C_1$-$C_2$, thio (-SH), alkyl($C_1$-$C_4$)sulfinyle, alkyl($C_1$-$C_4$)sulfonyle, thioalkyle($C_1$-$C_4$); de préférence un radical alkyle en $C_1$-$C_4$ éventuellement substitué par un radical hydroxy
- un groupement amino éventuellement substitué par un ou plusieurs radicaux alkyle en $C_1$-$C_4$, identiques ou différents, éventuellement porteurs d'au moins un groupement hydroxyle,
- un groupement alkylcarbonylamino (RCO-NR-) dans lequel les radicaux R indépendamment les uns des autres représentent un radical alkyle en $C_1$-$C_4$ ;
- un groupement alkylsulfonylamino ($RSO_2$-NR'-) dans lequel les radicaux R, R', indépendamment les uns des autres, représentent un atome d'hydrogène, un radical alkyle en $C_1$-$C_4$ ;
- un radical hydroxycarbonyle (-COOH)
- un radical alcoxycarbonyle en $C_1$-$C_2$
- un radical phényle éventuellement substitué par un ou plusieurs radicaux choisis parmi les radicaux hydroxy, alcoxy en $C_1$-$C_2$, (poly)-hydroxyalcoxy en $C_2$-$C_4$, amino, (di)alkylamino en $C_1$-$C_2$ ; de préférence un radical phényle éventuellement substitué par un radical choisi parmi les radicaux hydroxy, amino, ou leur combinaison
- un radical benzyle éventuellement substitué par un ou plusieurs radicaux choisis parmi les radicaux hydroxy, alcoxy en $C_1$-$C_2$, (poly)-hydroxyalcoxy en $C_2$-$C_4$, amino, (di)alkylamino en $C_1$-$C_2$ ; de préférence un radical benzyle éventuellement substitué par un radical choisi parmi les radicaux hydroxy, amino ou leur combinaison.

De façon encore plus préférée, le radical $R_6$ représente:

- un hydrogène,
- un radical méthyle, éthyle, 2-hydroxyéthyle,
- un radical amino,
- un radical hydroxycarbonyle
- un radical méthoxycarbonyle
- un groupement méthylcarbonylamino ($CH_3CO$-NH-) ;
- un groupement méthylsulfonylamino ($CH_3SO_2$-NH-);
- un radical phényle éventuellement substitué par un groupement hydroxy et/ou amino ou leur combinaison.
- un radical benzyle éventuellement substitué par un groupement hydroxy et/ou amino ou leur combinaison.

Encore plus particulièrement, $R_6$ représente un atome d'hydrogène.

Les composés polycationiques selon l'invention sont plus particulièrement choisis parmi les composés pour lesquels Z représente un radical alkylène en $C_2$-$C_{20}$:

1- interrompu par un ou plusieurs groupements, identiques ou non, correspondant aux formules suivantes :

| | |
|---|---|
| 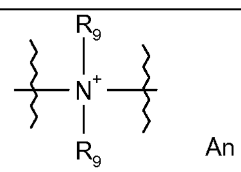 (a) | 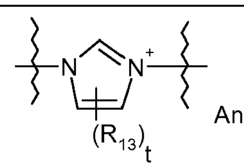 (b) |
| 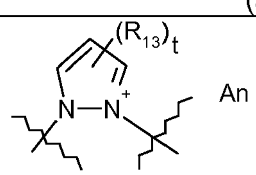 (c) | 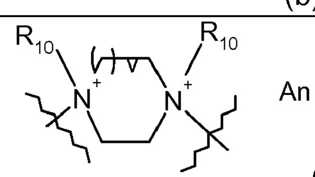 (d) |

| | |
|---|---|
| 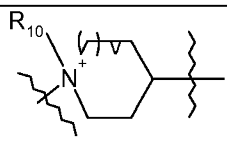 An (e) | 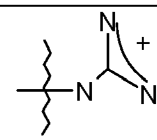 An (f) |

Dans lesquelles :

- $R_9$ et $R_{10}$ indépendamment l'un de l'autre, représentent un radical alkyle en $C_1$-$C_8$ ; un radical monohydroxyalkyle en $C_1$-$C_6$ ; un radical polyhydroxyalkyle en $C_2$-$C_6$ ; un radical alcoxy($C_1$-$C_6$)alkyle en $C_1$-$C_6$ ; un radical aryle tel que phényle éventuellement substitué; un radical arylalkyle tel que benzyle éventuellement substitué; un radical aminoalkyle en $C_1$-$C_6$ dont l'amine est substituée par un ou deux radicaux alkyle $C_1$-$C_4$ identiques ou différents; un radical alkyl($C_1$-$C_6$)sulfonyle ;
- deux radicaux $R_9$ peuvent former ensemble, avec l'atome d'azote auxquels ils sont rattachés, un cycle saturé ou insaturé, éventuellement substitué, à 5, 6 ou 7 chaînons ;
- $R_{13}$, identiques ou différents, représentent un atome d'hydrogène, un atome d'halogène choisi parmi le brome, le chlore ou le fluor, un radical alkyle en $C_1$-$C_6$, un radical monohydroxyalkyle en $C_1$-$C_6$, un radical polyhydroxyalkyle en $C_2$-$C_6$, un radical alcoxy en $C_1$-$C_6$, un radical (di-) alkylamino en $C_1$-$C_4$, un radical hydroxycarbonyle, un radical alkylcarbonyle en $C_1$-$C_6$, un radical thioalkyle en $C_1$-$C_6$, un radical alkyl($C_1$-$C_6$)thio, un radical alkyl($C_1$-$C_6$)sulfonyle, un radical benzyle éventuellement substitué, un radical phényle éventuellement substitué par un ou plusieurs radicaux choisis parmi les radicaux méthyle, hydroxyle, amino, méthoxy ;
- An est un anion ou un mélange d'anions, organiques ou minéraux ;
- t est un nombre entier compris entre 0 et 3 ; si t < 3, alors les atomes de carbone non substitués portent un atome d'hydrogène ;
- v est un entier égal à 1 ou 2 de préférence égal à 1 ;

2- et éventuellement interrompu par un ou plusieurs hétéroatomes ou groupements comprenant au moins un hétéroatome ou leurs combinaisons, comme par exemple l'oxygène, l'azote, le soufre, un groupement -CO-, un groupement $-SO_2-$ ; à la condition qu'il n'y ait pas de groupe ou liaison azo, nitro, nitroso, peroxo dans le groupement Z ;

3- et éventuellement substitué par un ou plusieurs radicaux choisis parmi les radicaux hydroxyle, alcoxy en $C_1$-$C_2$, (poly)-hydroxyalcoxy en $C_2$-$C_4$, amino substitué par un ou deux groupements alkyle linéaires en $C_1$-$C_2$ éventuellement porteurs d'au moins un groupement hydroxyle, trialkyl($C_1$-$C_4$)silyle, imidazole.

De préférence, Z représente un radical alkylène en $C_2$-$C_{20}$:

1-interrompu par un ou deux groupements identiques ou non, correspondant aux formules suivantes :

| | |
|---|---|
| 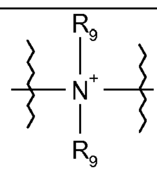 An (a) | 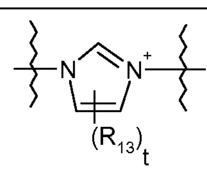 An (b) |

Dans lesquelles :

- $R_9$ indépendamment l'un de l'autre, représentent un radical alkyle en $C_1$-$C_8$ ; un radical monohydroxyalkyle en $C_1$-$C_6$ ; un radical alcoxy($C_1$-$C_6$)alkyle en $C_1$-$C_6$ ; un radical phényle ; un radical benzyle;
- deux radicaux $R_9$ peuvent former ensemble, avec l'atome d'azote auxquels ils sont rattachés, un cycle saturé ou insaturé, éventuellement substitué, à 5, 6 chaînons ;
- $R_{13}$, identiques ou différents, représentent un atome d'hydrogène, un radical alkyle en $C_1$-$C_6$, un radical monohydroxyalkyle en $C_1$-$C_6$, un radical alcoxy en $C_1$-$C_6$, un radical (di-) alkylamino en $C_1$-$C_4$ ;
- An est un anion ou un mélange d'anions cosmétiquement acceptables, organiques ou minéraux ;
- t est un nombre entier compris entre 0 et 3 ; si t < 3, alors les atomes de carbone non substitués portent un

atome d'hydrogène ;

- v est un entier égal à 1 ou 2, de préférence égal à 1 :

2- et éventuellement substitué par un radical choisi parmi les radicaux hydroxyle, alcoxy en $C_1$-$C_2$, hydroxyalcoxy en $C_2$-$C_4$, amino substitué par un ou deux groupements alkyle linéaires en $C_1$-$C_2$ éventuellement porteurs d'un groupement hydroxyle, trialkyl($C_1$-$C_3$)silyle, imidazole.

Selon une variante avantageuse de l'invention, les composés polycationiques selon l'invention sont tels que X représente $CR_2$, les radicaux $R_2$, identiques ou non, étant définis comme précédemment

Plus particulièrement, les composés polycationiques selon l'invention correspondent aux formules suivantes, leurs formes tautomères ainsi que leurs sels d'addition avec un acide et leurs solvates :

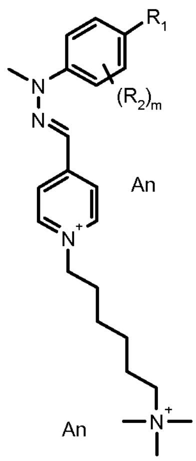
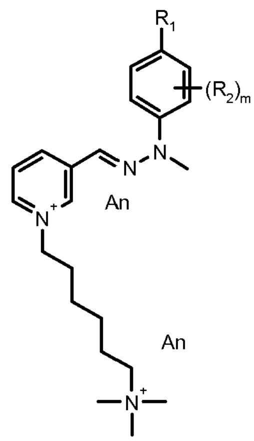
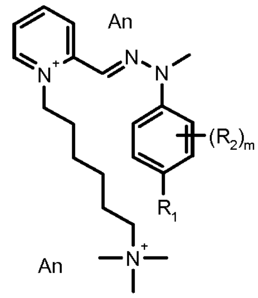
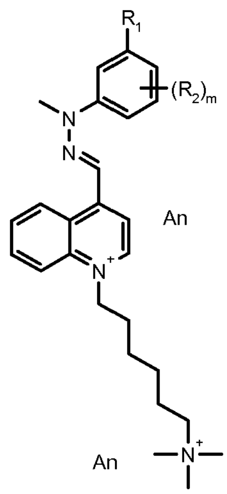

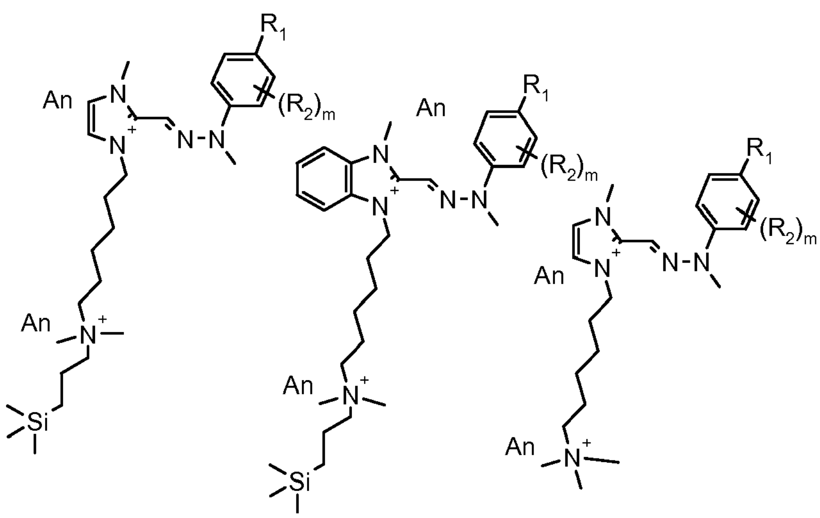
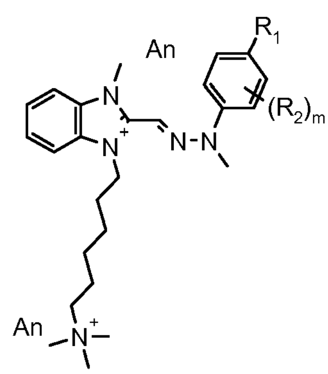

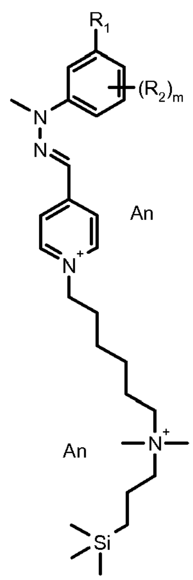
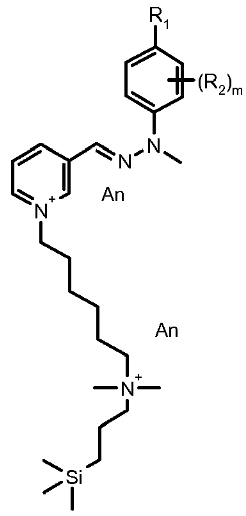
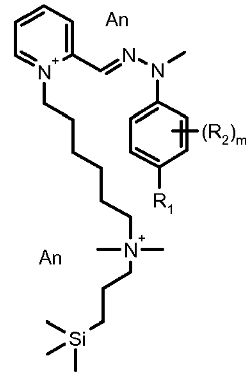
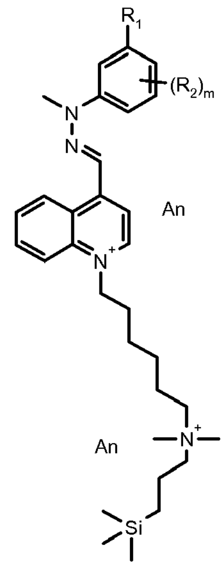

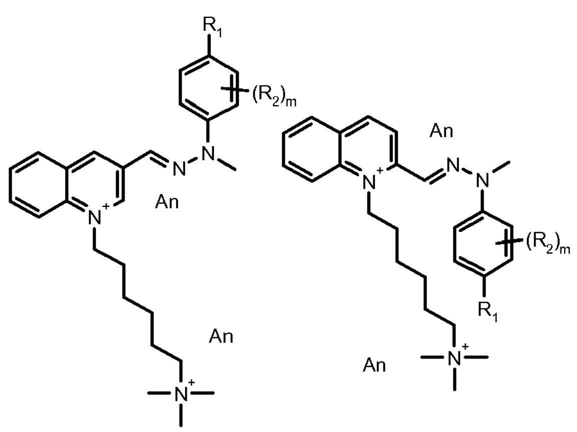
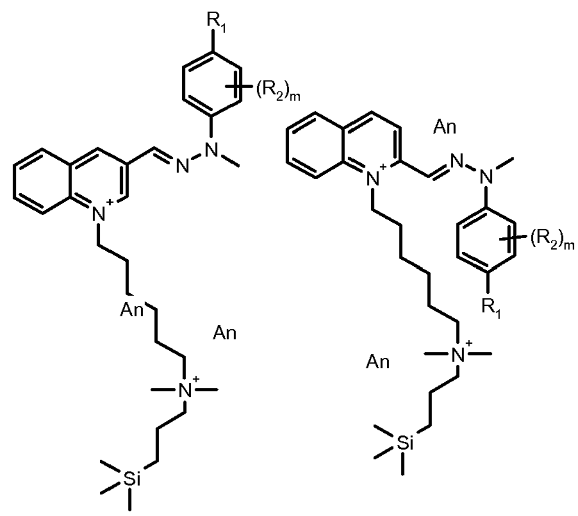

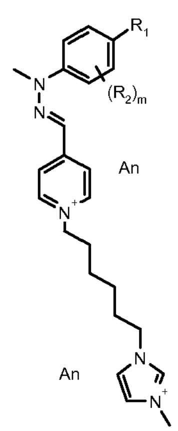
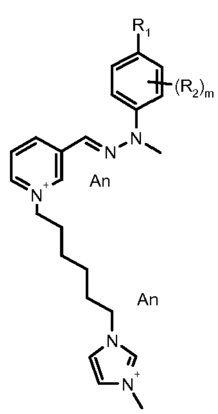
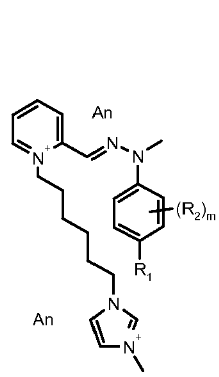
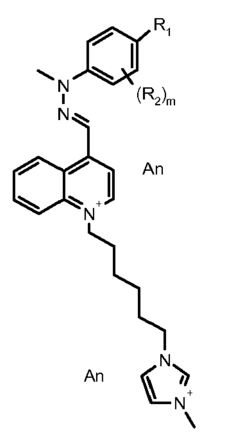

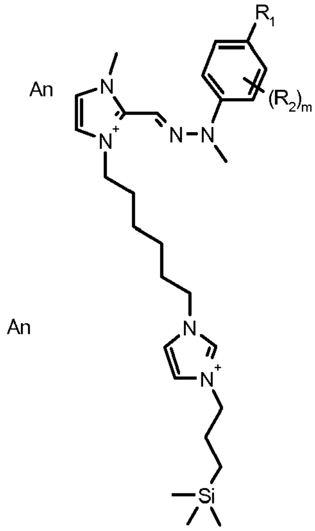
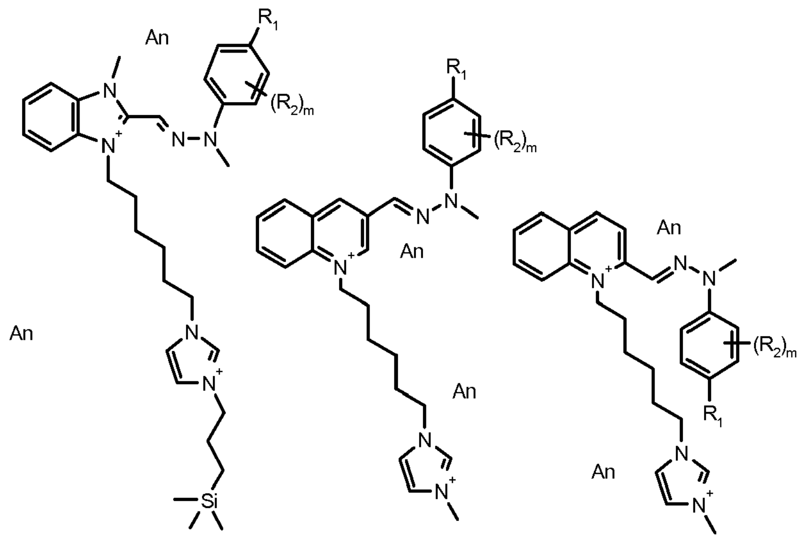

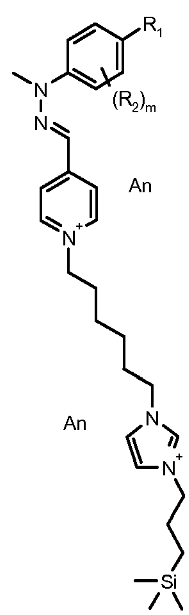
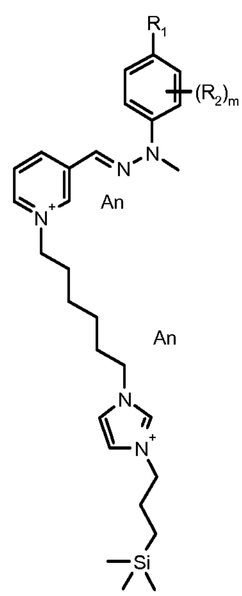
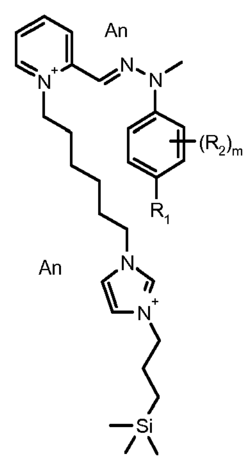
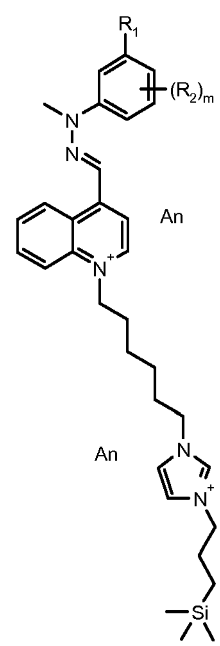

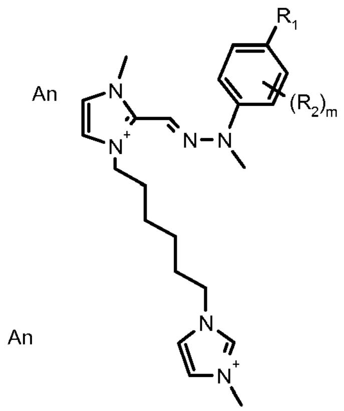

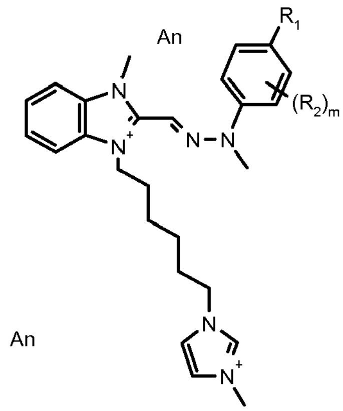

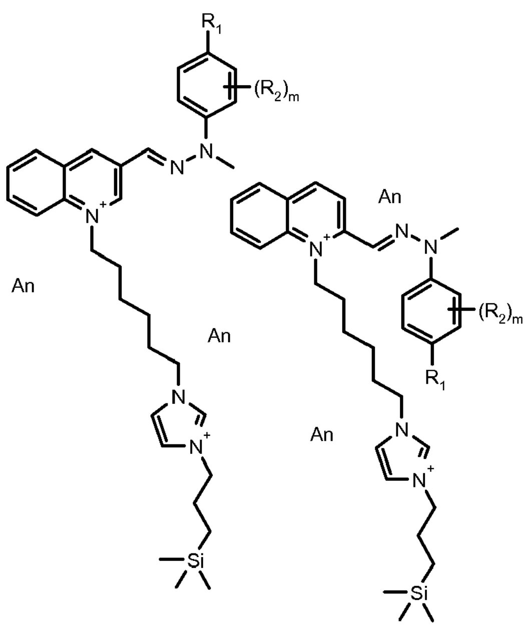

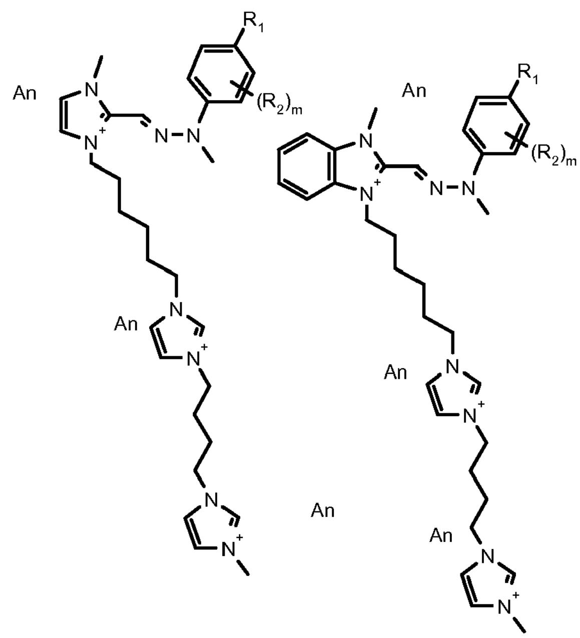

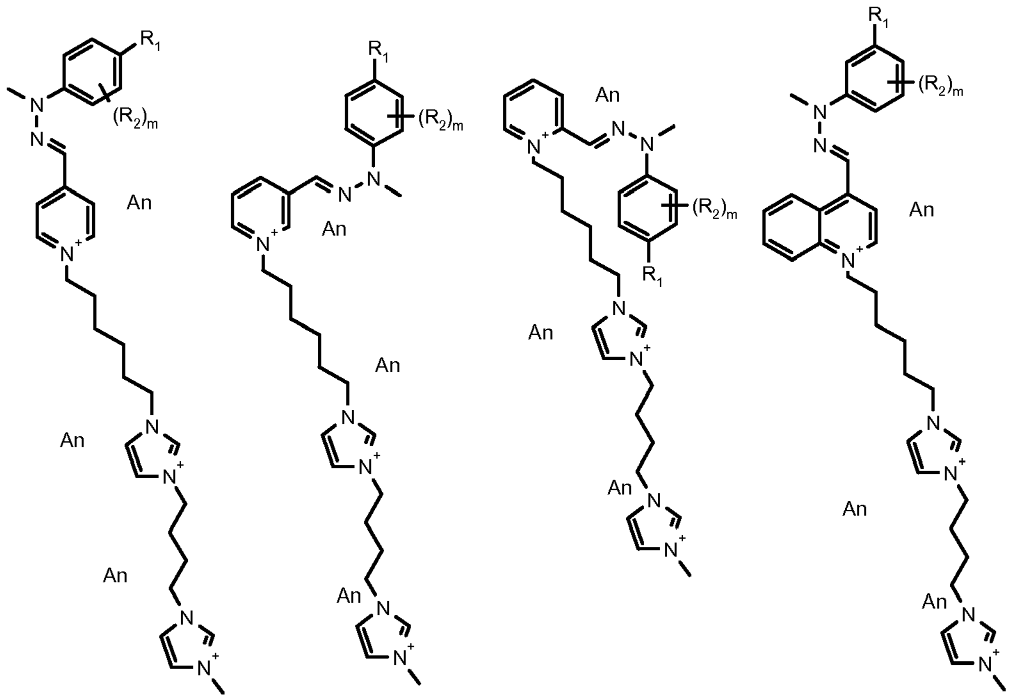

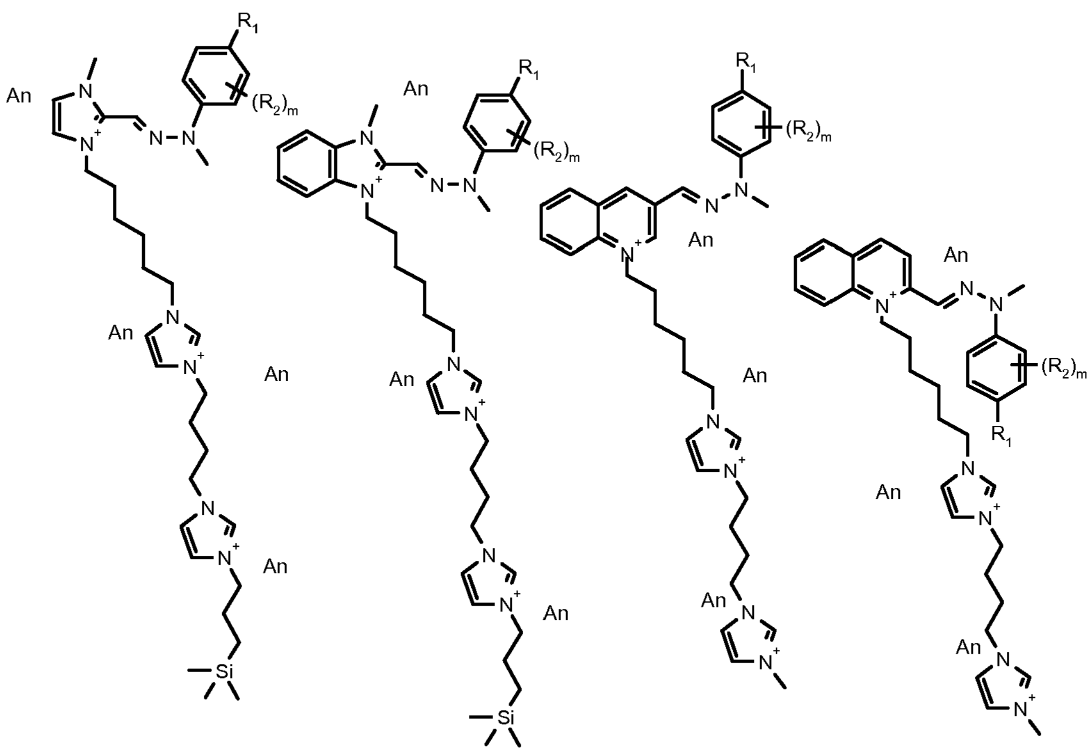

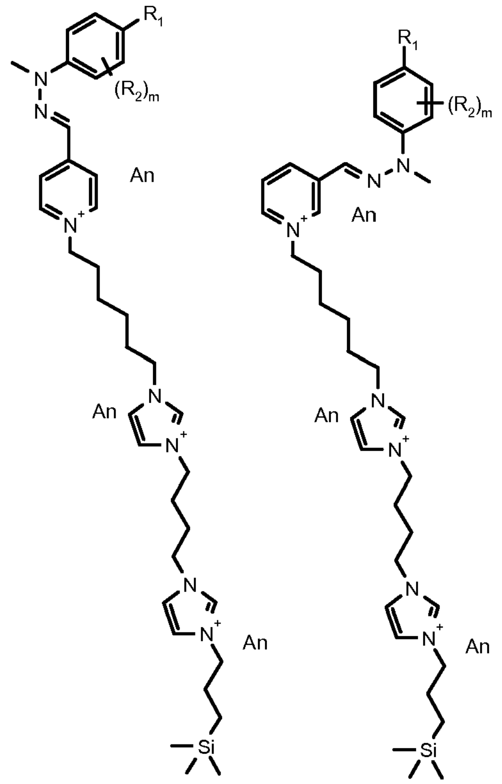

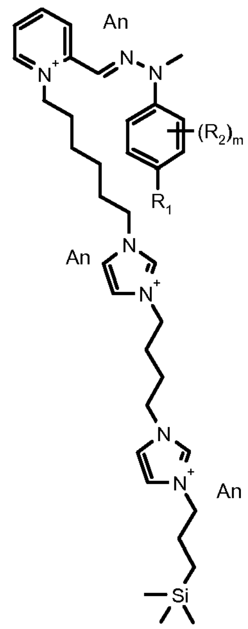

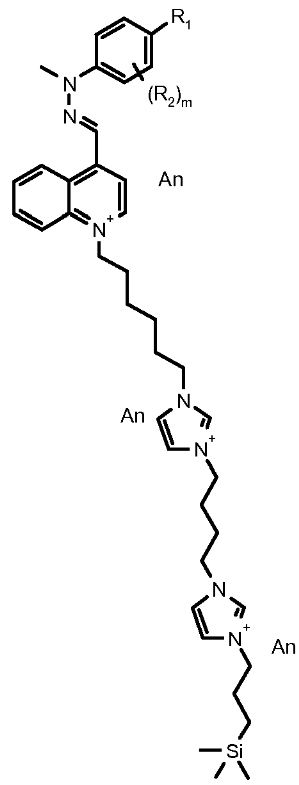

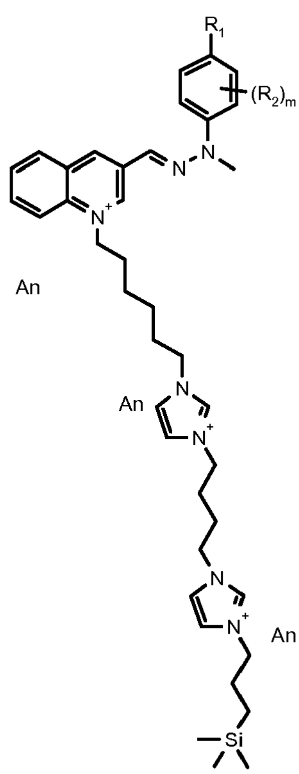

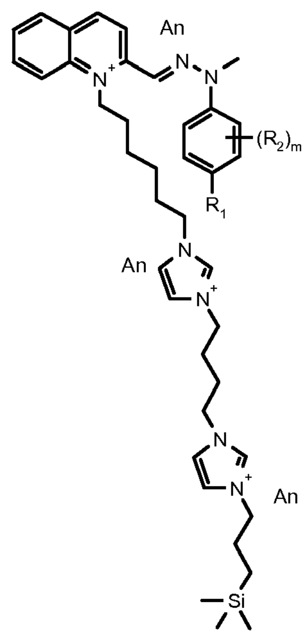

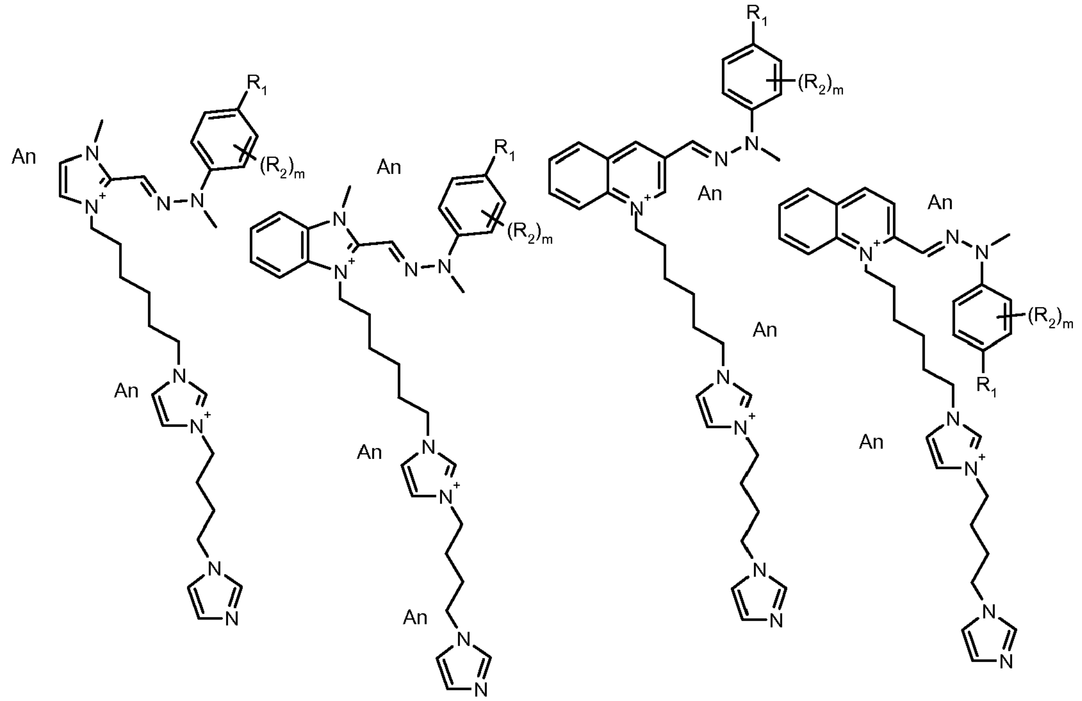

Formules dans lesquelles $R_1$, $R_2$, m, An ont les mêmes significations que celles indiquées précédemment, et R représente un atome d'hydrogène ou un radical méthyle.

Ces composés peuvent être synthétisés selon l'un des schémas réactionnels ci-dessous :

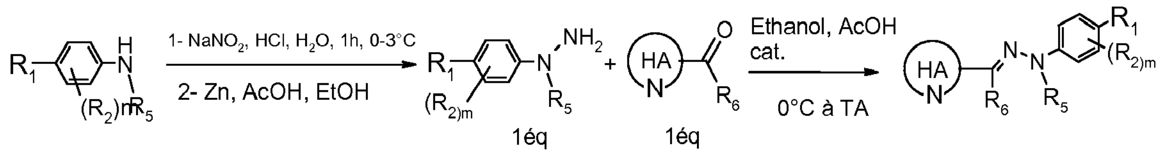


HA = hétérocycle
corespondant à W1

Synthèse d'hydrazone dicationique :

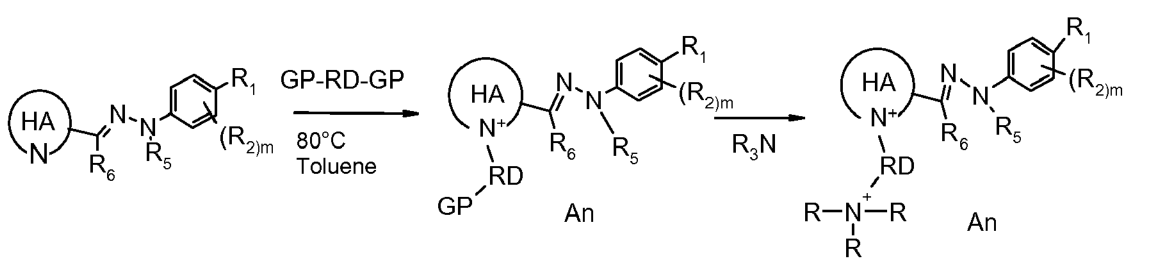


GP = groupe partant
RD = radical divalent

R identiques ou non, peuvent également former un hétérocycle saturé, insaturé ou aromatique à 5 ou 6 chaînons

Synthèse d'hydrazone dicationique :

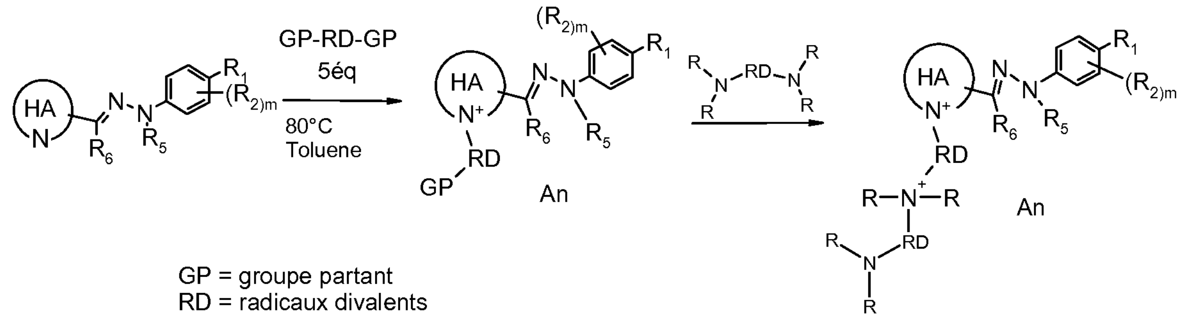


GP = groupe partant
RD = radicaux divalents identiques ou non

R identiques ou non, peuvent également former un hétérocycle saturé, insaturé ou aromatique à 5 ou 6 chaînons

Il est à noter que le radical divalent RD est choisi en fonction de la nature du groupement Z ; GP est en général un atome d'halogène comme le brome, le chlore, les groupements mésyle, tosyle ; An provient de ce groupe partant GP.

Pour obtenir un composé tricationique, on peut faire réagir de manière connue de l'homme du métier un composé comprenant un groupe partant comme par exemple des composés halogénés et le faire réagir avec le composé final de la deuxième synthèse de composé dicationique. On effectuerait de ce fait une quaternisation de la fonction amine terminale.

La première étape de synthèse de l'hydrazine, si ce composé n'est pas commercial, peut être réalisée de façon connue en faisant réagir dans une première étape l'amine correspondante avec du nitrite de sodium, en milieu aqueux acide. Dans une seconde étape, on ajoute un catalyseur à base de zinc, en présence d'acide acétique et d'éthanol.

Classiquement, cette réaction est réalisée à une température comprise entre 0˚C et 13˚C, de préférence entre 0 et 9˚C.

Il n'est pas nécessaire d'isoler le produit obtenu du milieu ractionnel.

On effectue ensuite une première étape de condensation d'un dérivé d'hydrazine sur un aldéhyde ou une cétone hétéroaromatique de manière connue de l'homme du métier.

La réaction est avantageusement effectuée en présence d'une quantité catalytique d'acide comme par exemple l'acide acétique, l'acide paratoluène sulfonique, l'acide citrique,...).

Habituellement cette réaction a lieu à une température comprise entre 0˚C et 100˚C de préférence entre 0˚C et 80˚C.

De manière classique, la réaction a lieu en présence d'un solvant approprié parmi lesquels on peut citer l'eau, les alcools tels que le méthanol ou l'éthanol ou l'isopropanol, le dichlorométhane, le diméthylformamide, le tétrahydrofurane, le 1,3-diméthyl-2-oxohexahydropyrimidine, la N-méthylpyrolidone, le sulfolane. On obtient ainsi un composé précurseur des motifs de formule (1) à (8).

On effectue ensuite une réaction de quaternisation du noyau hérérocyclique.

Plus particulièrement, cette étape consiste à faire réagir le produit résultant de l'étape précédente avec une quantité importante (en général 5 à 10 équivalents) d'un dérivé présentant deux groupements partants (noté GP-RD-GP sur le schéma réactionnel).

A titre d'exemple, ce dérivé peut être un α,ω-dihalogénoalkyle éventuellement substitué tel que le 1,6-dibromohexane ou le 1,5-dichloropentane ou le 1,5-diiodohexane, un dialkylsulfonate ou diarylsulfonate tel que le 4-[(methylsulfonyl)oxy]butyl methanesulfonate ou le 7-{[(4-methylphenyl)sulfonyl]oxyl} heptyl 4-methylbenzène sulfonate.

Habituellement cette réaction a lieu à une température comprise entre 50˚C et 130˚C de préférence entre 60˚C et 110˚C.

De manière classique, la réaction a lieu en l'absence ou en présence d'un solvant approprié tel que par exemple le 1,2-dichloroéthane, le toluène, le 3,4,5,6-térahydro-2(1 H)-pyrimidinone (DMPU), le diméthylformamide (DMF), la N-méthylpyrrolidone (NMP).

Dans la dernière étape on fait réagir le produit résultant en présence d'un nucléophile (amine tertiaire).

L'amine tertiaire, notée $NR_3$ dans le schéma réactionnel, est un groupement précurseur du groupement cationique de formule (a), (b), (c), (d), (e) ou (f), et peut enter autres correspondre à l'imidazole, au méthylimidazole, au 1-[4-(1H-imidazol-1-yl)butyl]-1 H-imidazole, à une amine tertiaire telle que la triméthylamine, la triéthylamine, la N-méthylpyrrolidine.

Habituellement cette réaction a lieu à une température comprise entre 50˚C et 130˚C de préférence entre 60˚C et 110˚C.

De manière classique, la réaction a lieu en présence d'un solvant approprié tel que par exemple le diméthylformamide, le butanol, le 3,4,5,6-térahydro-2(1H)-pyrimidinone (DMPU), la N-méthylpyrrolidone (NMP), le sulfolane.

A titre d'exemples plus particuliers mais non limitatifs, on peut aussi mettre en oeuvre les étapes suivantes, selon que l'on souhaite obtenir des composés di-, tri- ou tétra- cationiques à cations identiques ou non :

<u>Synthèse d'hydrazone dicationique :</u>

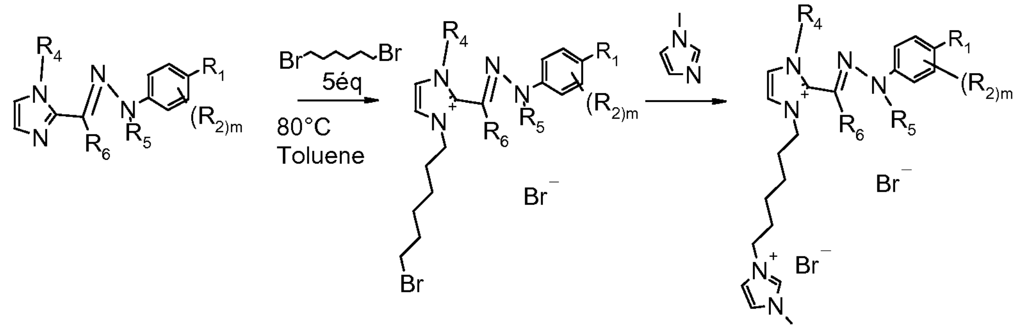


La même réaction peut être réalisée avec les amines suivantes :

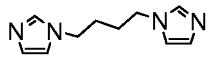 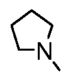 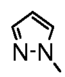 N(Et)$_3$ N(Me)$_3$,

Synthèse d'hydrazone tricationique à cation identique :

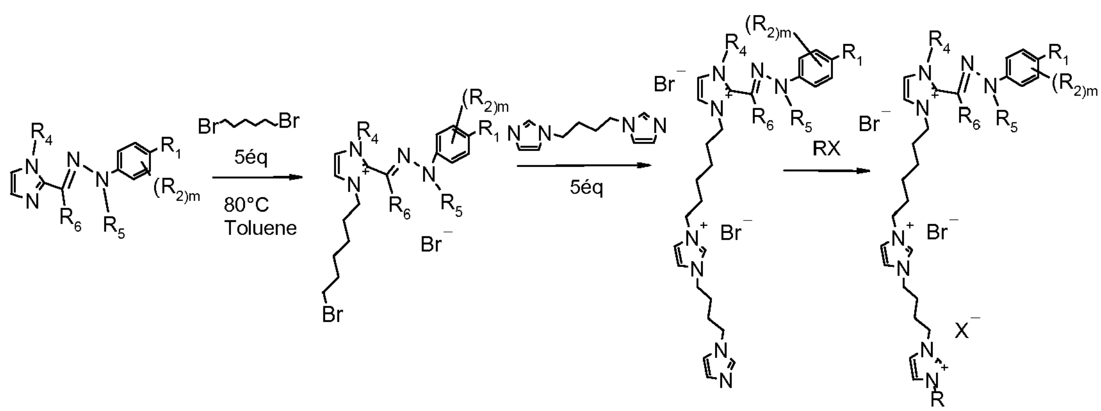


La même réaction peut être réalisée avec les amines suivantes (liste non exhaustif):

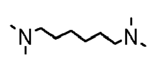 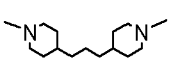 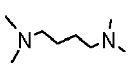 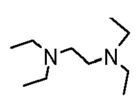

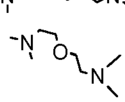 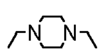

Synthèse d'hydrazone tri- et tétracationique à cation différent :

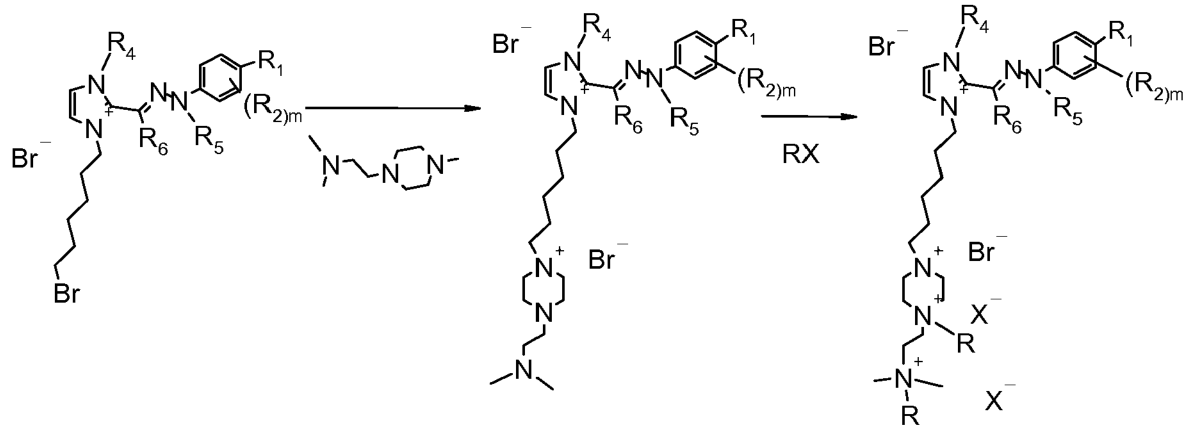


La même réaction peut être réalisée avec les amines suivantes :

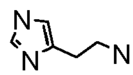

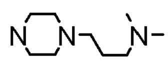

Un autre objet de la présente invention est constitué par une composition pour la coloration des fibres kératiniques humaines, en particulier les cheveux, comprenant dans un milieu cosmétiquement acceptable, un ou plusieurs composés polycationiques de formule (1) tels que décrits précédemment, en tant que colorant(s) direct(s).
Plus particulièrement, la composition selon l'invention comprend de 0,001 à 10%, de préférence 0,01 à 10%, en poids de composé(s) de formule (I), par rapport au poids total de la composition.
La composition tinctoriale conforme à l'invention peut en outre contenir un ou plusieurs colorants directs additionnels autres que les composés polycationiques de formule (I), ces colorants additionnels pouvant notamment être choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques, les colorants de type tétraazapentaméthinique et les colorants directs naturels.
Parmi les colorants directs benzéniques utilisables selon l'invention, on peut citer de manière non limitative les composés suivants :

- 1,4-diamino-2-nitrobenzène,
- 1-amino-2 nitro-4-ß- hydroxyéthylaminobenzène
- 1-amino-2 nitro-4-bis(β-hydroxyéthyl)-aminobenzène
- 1,4-Bis(ß -hydroxyéthylamino)-2-nitrobenzène
- 1-ß-hydroxyéthylamino-2-nitro-4-bis-(β-hydroxyéthylamino)-benzène
- 1-ß-hydroxyéthylamino-2-nitro-4-aminobenzène
- 1-β-hydroxyéthylamino-2-nitro-4-(éthyl)(ß-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène

- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1- β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO-95/01772 et EP-714954 dont le contenu fait partie intégrante de l'invention.

Parmi ces composés on peut tout particulièrement citer les colorants suivants:

- sel de de 1,3-diméthyl-2-[[4-(diméthylamino)phényl]azo]-1H-Imidazolium, notamment chlorure,
- sel de 1,3-diméthyl-2-[(4-aminophényl)azo]-1H-Imidazolium, notamment chlorure,
- sel de 1-méthyl-4-[(méthylphénylhydrazono)méthyl]-pyridinium, notamment méthylsulfate.

On peut également citer parmi les colorants directs azoïques les colorants suivants, décrits dans le COLOUR INDEX INTERNATIONAL 3e édition :

- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :

- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7

- Basic Blue 22
- Disperse Violet 15
- Basic Blue 99

ainsi que les composés suivants:

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :

- Basic Blue 17
- Basic Red 2.

Parmi les colorants triarylméthaniques utilisables selon l'invention, on peut citer les composés suivants :

- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :

- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants : le chlorure de 2-((E)-{(E)-[(1,3-dimethyl-1,3-dihydro-2H-imidazol-2-ylidene)hydrazono]methyl}diazenyl)-1,3-dimethyl-1H-imidazol-3-ium, le chlorure de 2-{(E)-[(1Z)-N-(1,3-dimethyl-1,3-dihydro-2H-imidazol-2-ylidene)ethane hydrazonoyl]diazenyl}-1,3-dimethyl-1H-imidazol-3-ium, le chlorure de 4-methoxy-2-((E)-{(1 E)-1-[(2E)-(4-methoxy-1-methylpyridin-2(1 H)-ylidene)hydrazono]ethyl}diazenyl)-1-methylpyridinium, le chlorure de 1-methyl-2-((E)-{(1E)-1-[(2E)-(1-methylpyridin-2(1H)-ylidene)hydrazono]ethyl}diazenyl)pyridinium, le chlorure de 1-(2-hydroxyethyl)-2-[(E)-((1E)-1-{(2E)-[1-(2-hydroxyethyl)pyridin-2(1H)-ylidene]hydrazono}ethyl)diazenyl] pyridinium, le chlorure de 1-methyl-2-((E)-{(E)-[(2Z)-(1-methylpyridin-2(1H)-ylidene) hydrazono]methyl}diazenyl)pyridinium, l'acétate de 1-(2-hydroxyethyl)-2-[(E)-((E)-{(2E)-[1-(2-hydroxyethyl)pyridin-2(1H)-ylidene]hydrazono}methyl)diazenyl]pyridinium.

Parmi les colorants directs additionnels naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs additionnels représentent de préférence de 0,001 à 20% en poids environ du poids total de la composition prête à l'emploi et encore plus préférentiellement de 0,005 à 10% en poids environ.

La composition de la présente invention peut en outre comprendre au moins une base d'oxydation en mélange avec au moins un coupleur, précurseurs de colorant d'oxydation..

A titre d'exemple, si elle(s) est (sont) présente(s), chaque, base d'oxydation peut être choisie parmi les paraphénylènediamines, les bis-phénylalkylènediamines, les para-aminophénols, les ortho-aminophénols, les bases hétérocycliques, ainsi que son (leurs) sel(s) d'addition avec un acide.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a] pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;

ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino

1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation additionnelles éventuellement présentes dans la composition de l'invention sont en général présentes en quantité comprise allant de 0,001 à 20 % en poids environ du poids total de la composition tinctoriale, de préférence allant de 0,005 à 6 %.

Si la composition selon l'invention contient au moins une base d'oxydation additionnelle, elle contient en outre un ou plusieurs coupleurs conventionnellement utilisés pour la teinture de fibres kératiniques. Parmi ces coupleurs, on peut notamment citer les métaphénylènediamines, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthyl-amino)toluène et leurs sels d'addition.

Dans la composition de la présente invention, s'ils sont présents, le ou les coupleurs sont généralement présents en quantité comprise allant de 0,001 à 20 % en poids environ du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les acides alkylsulfoniques dont la partie alkyle, linéaire ou ramifiée, est en C1-C6 comme l'acide méthylsulfonique par exemple, les citrates, les succinates, les tartrates, les lactates, les tosylates, les acides arylsulfoniques éventuellement substitués tels que par exemple l'acide benzènesulfonique ou l'aide para-toluènesulfonique,, les phosphates et les acétates.

Le milieu cosmétiquement acceptable est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale selon l'invention peut également renfermer divers adjuvants utilisés classiquement dans les compositions pour la teinture des cheveux, tels que des agents tensioactifs anioniques, cationiques, non ioniques, amphotères, zwitterioniques ou leurs mélanges, des polymères anioniques, cationiques, non ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

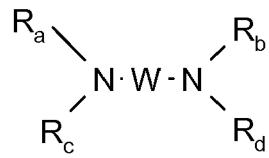

(II)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, de pâtes ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment.

Selon un mode de réalisation particulier, la composition de l'invention est appliquée sur les fibres kératiniques en présence d'un agent oxydant pendant un temps suffisant pour obtenir l'éclaircissement souhaité.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention comprend au moins un précurseur de colorant d'oxydation.

Le mélange obtenu est ensuite appliqué sur les fibres kératiniques.

Après un temps de pause compris entre 3 minutes et 1 heure et de préférence entre 5 et 50 minutes, les fibres kératiniques sont de préférence rincées à l'eau, éventuellement lavées au shampooing, rincées à l'eau à nouveau puis séchées.

Les agents oxydants classiquement utilisés pour la coloration des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Selon un mode de réalisation particulier, la composition contient un agent oxydant de type peroxyde et/ou un agent oxydant de type persels, par exemple un mélange peroxyde d'hydrogène et persulfates, le peroxyde d'hydrogène seul ou le persulfate seul. Conformément à un mode de réalisation particulièrement avantageux, l'agent oxydant est le peroxyde d'hydrogène seul.

La composition oxydante peut également renfermer divers adjuvants utilisés classiquement dans les compositions pour la teinture des cheveux et tels que définis précédemment.

Un autre objet de l'invention est représenté par un dispositif à plusieurs compartiments, comprenant un premier compartiment renfermant une composition tinctoriale telle que définie précédemment et un second compartiment renfermant une composition comprenant au moins un agent oxydant.

Selon une variante, le dispositif comprend un premier compartiment renfermant une composition comprenant un ou plusieurs colorants polycationiques de formule (I) mais pas de précurseur de colorant d'oxydation (base, coupleur), un second compartiment comprenant une composition comprenant au moins un précurseur de colorant d'oxydation choisi parmi une ou plusieurs bases d'oxydation éventuellement associées à un ou plusieurs coupleurs, et un troisième compartiment renfermant une composition comprenant au moins un agent oxydant.

Ce qui a été indiqué à propos de la nature des colorants et des ingrédients utilisables dans ce domaine reste valable et l'on pourra se reporter aux parties correspondantes de la description. Ainsi, les compositions des premier, deuxième et troisième compartiments peuvent comprendre chacune les additifs classiques des compositions tinctoriales utilisables pour la coloration de fibres kératiniques humaines.

Les exemples suivants servent à illustrer l'invention sans toutefois présenter un caractère limitatif.

## EXEMPLE 1

**Synthèse du composé [5]** : Synthèse du dibromure de 1-(6-{3-[4-(1H-imidazol-1-yl)butyl]-1H-imidazol-3-ium-1-yl} hexyl)-4-{(E)-[methyl(phenyl)hydrazono]methyl}quinolinium

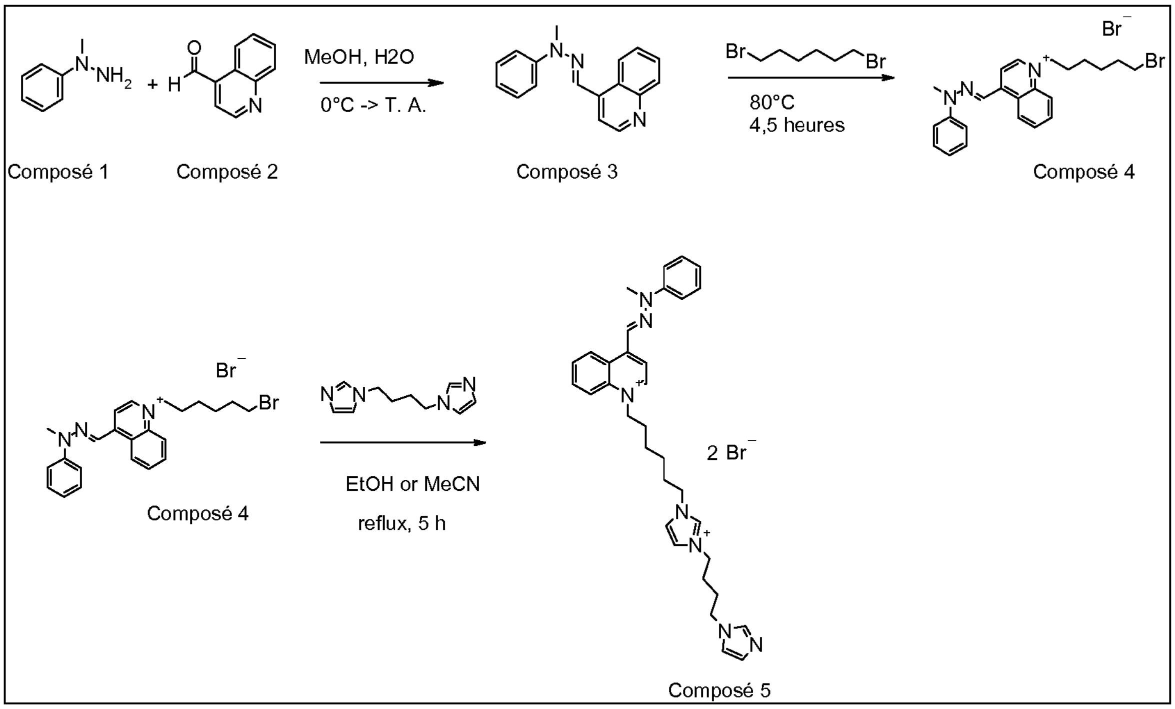


NB : les composés 1 et 2 sont commerciaux.

Etape 1

Dans un tricol de 250mL muni d'un thermomètre et surmonté d'un réfrigérant, on met sous agitation à 0˚C (température du milieu réactionnel) 4,7 ml (40 mmoles) de 1-methyl-1-phenylhydrazine [1] dans 8 ml d'eau. 6,8 g (40 mmoles) de 4-quinolinecarboxaldehyde [2] dans 40 ml de méthanol sont ensuite ajoutés lentement en maintenant le milieu réactionnel à 0˚C. Celui-ci est ensuite ramené lentement à température ambiante. La réaction est ensuite laissée 3 heures à température ambiante, temps pendant lequel un précipité jaune apparait.

Le précipité formé est ensuite filtré sous vide, lavé 3 fois à l'eau puis séché sous vide poussé. 10,3 g d'une poudre jaune correspondant au composé [3] sont obtenus.

Les spectres RMN et les spectres de Masse sont conformes au produit [3] attendu.

Etape 2

Dans un tricol de 250mL muni d'un thermomètre et surmonté d'un réfrigérant, on met sous agitation à 80˚C, 10 ml de 1,6-dibromohexane en présence de 0,5 g de composé [3]. Après 4,5 heures de réaction, le milieu réactionnel de couleur orange foncée est ensuite ramené à température ambiante. Un précipité se forme progressivement au cours du refroidissement.

Celui-ci est ensuite filtré sous vide, lavé au toluène puis séché sous vide poussé. 0,775 g d'une poudre orange correspondant au composé [4] sont obtenus.

Les spectres RMN et les spectres de Masse sont conformes au produit [4] attendu.

Etape 3

Dans un tricol de 250mL muni d'un thermomètre et surmonté d'un réfrigérant, on met sous agitation à reflux, l'intermédiaire [4] (4,5 g, 9 mmoles), 17 g de 1,4-bis(1-imidazolyl)butane (90 mmoles) solubilisés dans 90 ml d'acétonitrile. Après 7 heures de reflux, le mélange réactionnel est refroidi puis concentré ; le résidu obtenu est ensuite purifié par chromatographie sur colonne d'alumine pour fournir 2,5 g de composé [5].

Les spectres RMN et les spectres de Masse sont conformes au produit [5] attendu.

**EXEMPLE 2**

**Synthèse du composé [9] :** Synthèse du di bromure de 1-(6-{3-[4-(1 H-imidazol-1-yl)butyl]-1 H-imidazoi-3-ium-1-yl} hexyl)-2-{(E)-[methyl(phenyl)hydrazono]methyl}quinolinium

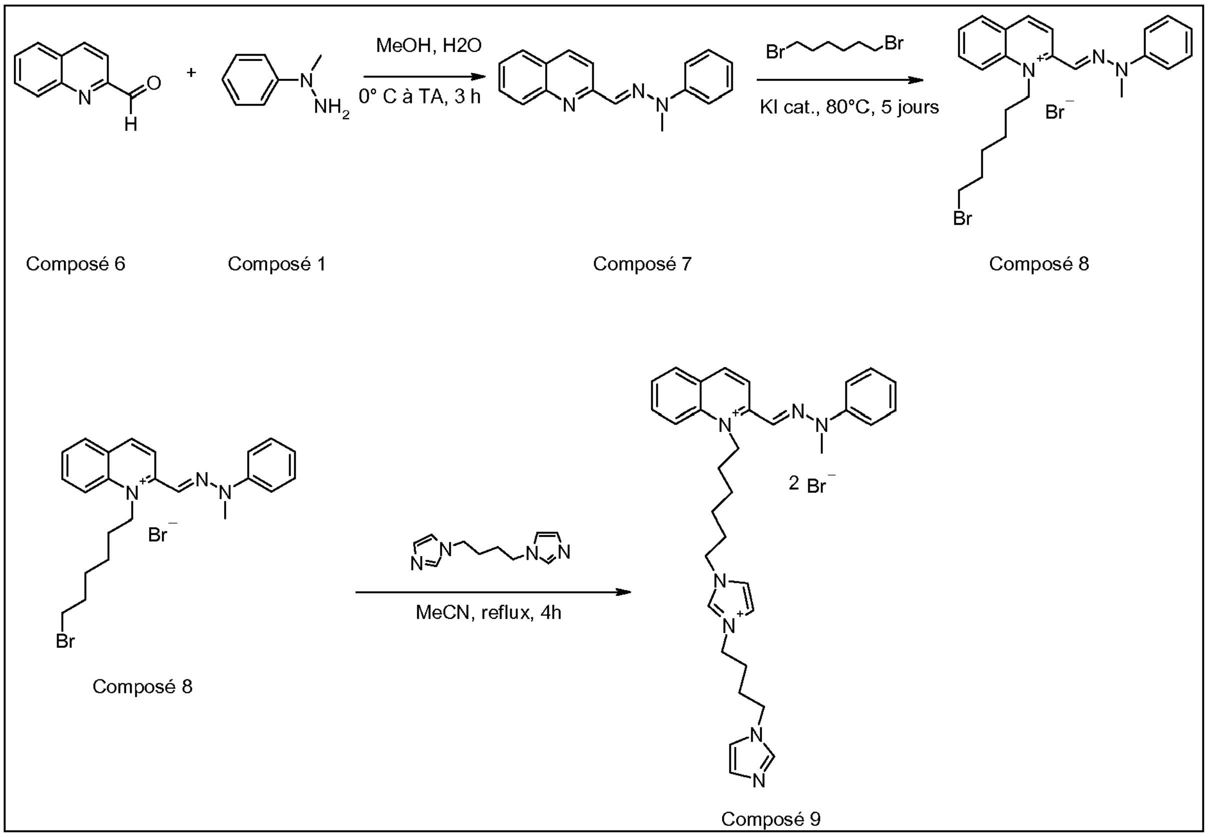


NB : le composé 6 est un composé commercial.

Etape 1

Dans un tricol de 250mL muni d'un thermomètre et surmonté d'un réfrigérant, on met sous agitation à 0˚C, 4,7 ml (40 mmoles) de 1-methyl-1-phenylhydrazine [1] dans 8 ml d'eau. Par la suite, 6,8 g (40 mmoles) de 2-quinolinecarboxaldehyde [6] solubilisés dans 40 ml de méthanol sont ensuite ajoutés lentement. Par la suite, le milieu réactionnel est mis sous agitation pendant 3 heures à température ambiante ; Un précipité se forme progressivement au cours de la réaction ; Celui-ci est ensuite filtré sous vide, nettoyé trois fois à l'eau puis séché sous vide poussé. 10,3 g du composé [7] sont obtenus.

Les spectres RMN et les spectres de Masse sont conformes au produit [7] attendu.

Etape 2

Dans un tricol de 250mL muni d'un thermomètre et surmonté d'un réfrigérant, on met sous agitation à 80˚C pendant 5 jours, 5.22 g (20 mmol) de l'intermédiaire [7] solubilisé dans 20 ml de 1,6-dibromohexane en présence d'une quantité catalytique de KI (60 mg). Après réaction, le milieu réactionnel est refroidi à température ambiante. Un précipité se forme progressivement au cours du refroidissement. Celui-ci est ensuite filtré sous vide, nettoyé à l'acétate d'éthyle puis séché. Après purification sur colonne de silice flash 0,8 g du composé [8] sont obtenus.

Les spectres RMN et les spectres de Masse sont conformes au produit L] attendu.

Etape 3

Dans un tricol de 250mL muni d'un thermomètre et surmonté d'un réfrigérant, on met sous agitation à reflux, l'intermédiaire [8] (1,6 g, 3,2 mmoles), 6 g de 1,4-bis(1-imidazolyl)butane (32 mmoles) solubilisés dans 20 ml d'acétonitrile. Après 4 heures de reflux, le mélange réactionnel est refroidi puis concentré ; le résidu obtenu est ensuite purifié par chromatographie sur colonne d'alumine (MeOH / $CH_2Cl_2$ = 1/100 à 1/10) pour fournir 1,2 g de composé [9].

Les spectres RMN et les spectres de Masse sont conformes au produit [9] attendu.

**EXEMPLE 3**

**Synthèse du composé [13]** : Synthèse du dibromure de 1-(6-{3-[4-(1H-imidazol-1-yl)butyl]-1H-imidazot-3-ium-1-yl} hexyl)-2-{(E)-[methyl(phenyl)hydrazono]methyl}pyridinium.

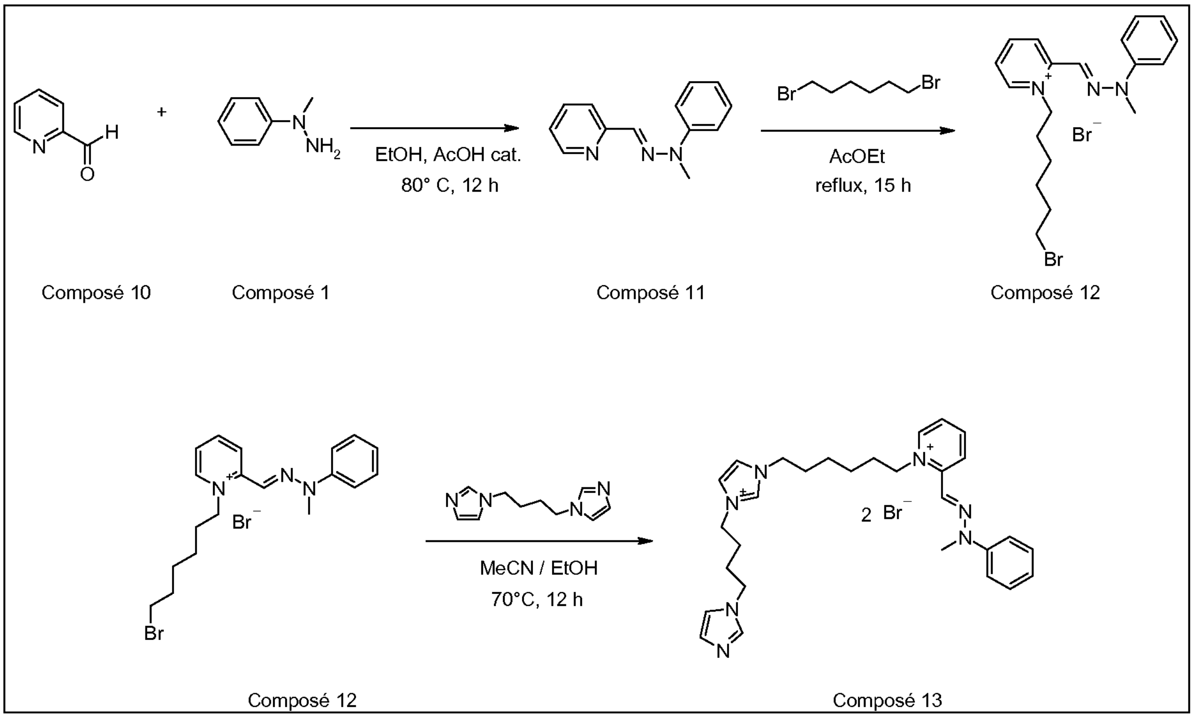


NB : le composé 10 est un composé commercial.

Etape 1

Dans un tricol de 250mL muni d'un thermomètre et surmonté d'un réfrigérant, on met sous agitation à 80°C pendant 12 h., 1,07 g (10 mmoles) de 2-pyridinecarboxaldehyde [10], 1,22 g (10 mmoles) de 1-methyl-1-phenylhydrazine [1] dans 20 ml d'éthanol en présence de 3 gouttes d'acide acétique.

Après concentration du milieu réactionnel, un résidu est obtenu; celui-ci est neutralisé jusqu'à pH 8 avec une solution saturée de $NaHCO_3$ puis extrait trois fois avec de l'acétate d'ethyle. Les phases organiques sont rassemblées puis séchées sur sulfate de magnésium, filtrées, et évaporées pour fournir 2 g de produit [11].

Les spectres RMN et les spectres de Masse sont conformes au produit [11] attendu.

Etape 2

Dans un tricol de 250mL muni d'un thermomètre et surmonté d'un réfrigérant, on met sous agitation à 80°C pendant 15 h., 2,1 g (10 mmoles) d'intermédiaire [11] et 24,4 g (100 mmoles) de 1,6-dibromohexane dans 15 ml d'acétate éthyle. Le milieu réactionnel de couleur jaune foncé est ensuite ramené à température ambiante. Un précipité se forme

progressivement au cours du refroidissement. Celui-ci est ensuite filtré sous vide, nettoyé à l'acétate d'éthyle puis séché. Après purification sur colonne de silice flash (méthanol/ dichlorométhane: 1/30 à 1/10), 2, 5 g d'une poudre jaune foncé correspondant au composé [12] sont obtenus.

Les spectres RMN et les spectres de Masse sont conformes au produit [12] attendu.

Etape 3

Dans un tricol de 250mL muni d'un thermomètre et surmonté d'un réfrigérant, on met sous agitation à 70°C, l'intermédiaire [12] (1,5 g, 2,7 mmoles), 5,1 g de 1,4-bis(1-imidazolyl)butane (27 mmoles) solubilisés dans un mélange constitué de 20 ml d'acétonitrile et 5 ml d'éthanol. Après 12 heures de reflux, le mélange réactionnel est refroidi puis concentré ; le résidu obtenu est ensuite purifié par chromatographie sur colonne d'alumine (MeOH / $CH_2Cl_2$ = 1/100 à 1/10) pour fournir 1,1 g de composé [13].

Les spectres RMN et les spectres de Masse sont conformes au produit [13] attendu.

## EXEMPLE 4

**Synthèse du composé [17] :** Synthèse du dibromure de 1-(6-{3-[4-(1H-imidazol-1-yl)butyl]-1H-imidazol-3-ium-1-yl} hexyl)-4-{(E)-[methyl(phenyl)hydrazono]methyl}pyridinium

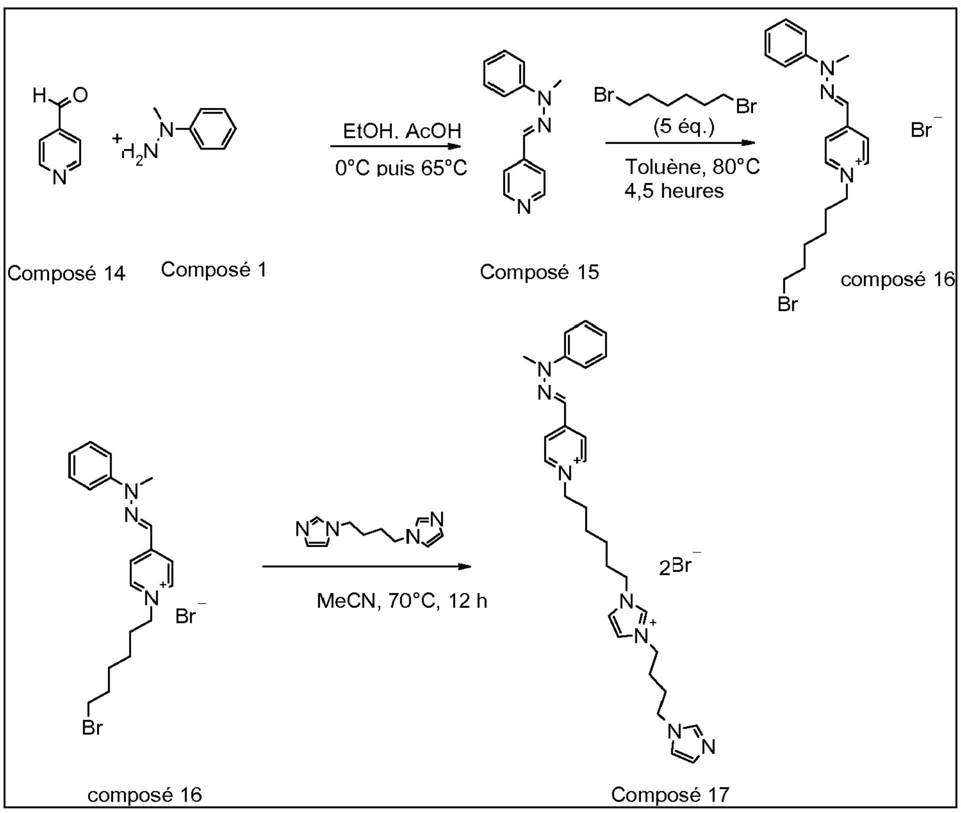


NB : le composé 14 est un composé commercial.

Etape 1

Dans un tricol surmonté d'un réfrigérant, on dilue un équivalent de N-méthylphénylhydrazine (58,76g ; 0,4809 mol, composé [1]) dans 50ml d'éthanol et on ajoute 1,5ml d'acide acétique. Le milieu réactionnel est alors mis sous agitation à 0°C. A l'aide d'une ampoule à brome, on ajoute 1 équivalent de 4-pyridinecarboxaldéhyde (51,5g ; 0,4808 mol, composé [14]). Après ajout de l'aldéhyde, on chauffe le milieu réactionnel à 65°C (température du bain d'huile) pendant 13 heures.

Par la suite, le milieu réactionnel est versé dans un mélange eau-glace (1/3). On place alors le mélange sous agitation. Après agitation, le résidu huileux conduit à l'obtention d'une poudre jaune. Celle-ci est alors filtrée puis lavée plusieurs fois à l'eau. Après séchage, on obtient 83,9g du composé [15].

Les spectres RMN et les spectres de Masse sont conformes au produit [15] attendu.

Etape 2

Dans un tricol de 3 litres surmonté d'un réfrigérant, on dilue le composé [15] (80,1 g, 0.379 mole) dans 250 ml de toluène. On porte à 80°C et on additionne en 10 minutes une solution de 1,6-dibromohexane (1.94 moles) dans 750 ml de toluène. Un précipité se forme au cours de la réaction. Après 4,5 heures de réaction, le milieu réactionnel est refroidi puis filtré.

Purification : Le précipité obtenu est lavé avec 200 ml de toluène puis 100 ml d'éther de pétrole. Par la suite, ce précipité est solubilisé dans 1 litre de dichlorométhane puis extrait à l'eau. La phase organique est lavée 4 fois à l'eau, séchée sur sulfate de magnésium, filtrée puis concentrée. On obtient une huile marron qui est reprise par 50 ml de toluène. On obtient un précipité jaune qui est séparé par filtration puis séché pour conduire à 95,1 g du composé [16] sous la forme d'une poudre jaune.

Les spectres RMN et les spectres de Masse sont conformes au produit [16] attendu.

Etape 3

Dans un tricol de 250mL muni d'un thermomètre et surmonté d'un réfrigérant, on met sous agitation à reflux, l'intermédiaire [16] (1.6 g, 3 mmoles), 5.7 g de 1,4-bis(1-imidazolyl)butane (30 mmoles) solubilisés dans 50 ml d'acétonitrile. Après 12 heures de reflux, le mélange réactionnel est refroidi puis concentré ; le résidu obtenu est ensuite purifié par chromatographie sur colonne d'alumine (MeOH / $CH_2Cl_2$ = 1/100 à 1/10) pour fournir 1,05 g de composé [17].

Les spectres RMN et les spectres de Masse sont conformes au produit [17] attendu.

**EXEMPLE 5**

**Synthèse du composé [21]** : synthèse du dibromure de 3-(6-{3-[4-(1H-imidazol-1-yl)butyl]-1H-imidazol-3-ium-1-yl} hexyl)-1-methyl-2-{(E)-[methyl(phenyl)hydrazono]methyl}-1 H-imidazol-3-ium

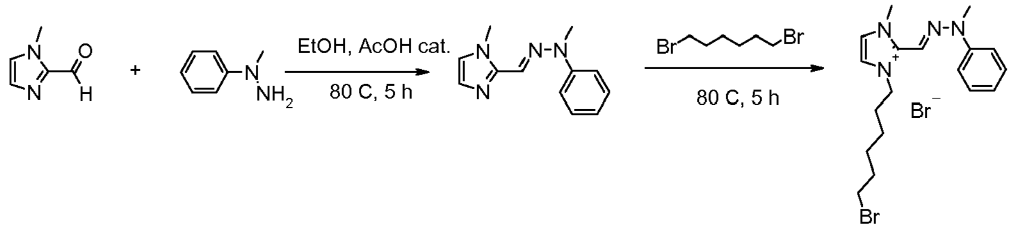


Composé 18 Composé 1 Composé 19 Composé 20

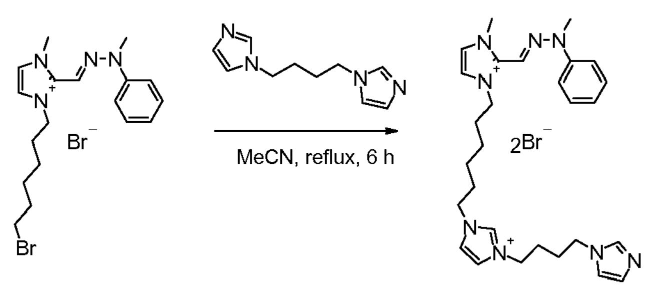


Composé 20 Composé 21

NB : le composé 18 est un composé commercial.

Etape 1

Dans un tricol de 250mL muni d'un thermomètre et surmonté d'un réfrigérant, on met sous agitation à 80˚C, 2,2 g (20 mmoles) de 1-methyl-2-imidazolecarboxaldehyde [18] et 2,44 g (20 mmoles) 1-methyl-1-phenylhydrazine [1] dans 50 ml d'éthanol en présence de 3 gouttes d'acide acétique. Après 5 heures de réaction, le milieu est ramené à température ambiante et le solvant est concentré ; la poudre obtenu est nettoyée avec de l'éther diéthylique. Après séchage, 4 g du composé [19] sont obtenus.

Les spectres RMN et les spectres de Masse sont conformes au produit [19] attendu.

Etape 2

Dans un tricol de 250mL muni d'un thermomètre et surmonté d'un réfrigérant, on met sous agitation à 80˚C 1,1g (5 mmoles) d'intermédiaire [19] et 12 ml (80 mmoles) de 1,6-dibromohexane. Après 5 heures de chauffage, le milieu réactionnel est refroidi à température ambiante. Un précipité jaune se forme progressivement au cours du refroidissement. Celui-ci est ensuite filtré, puis nettoyé avec de l'acétate d'éthyle et enfin sous vide poussé pour fournir 2,1 g de composé [20].

Les spectres RMN et les spectres de Masse sont conformes au produit [20] attendu.

Etape 3

Dans un tricol de 250mL muni d'un thermomètre et surmonté d'un réfrigérant, on met sous agitation à reflux, l'intermédiaire [20] (1,2 g, 2,6 mmoles), 5 g de 1,4-bis(1-imidazolyl)butane (26 mmoles) solubilisés dans 70 ml d'acétonitrile. Après 6 heures de reflux, le mélange réactionnel est refroidi puis concentré ; le résidu obtenu est ensuite solubilisé dans l'eau puis extrait au dichlorométhane. La phase aqueuse est concentrée. La poudre obtenue est purifié par chromatographie sur colonne d'alumine (MeOH / $CH_2Cl_2$ = 1/100 à 1/20) pour fournir 1,02 g de composé [21].

Les spectres RMN et les spectres de Masse sont conformes au produit [21] attendu.

## EXEMPLE 6

**Synthèse du composé [25]** : synthèse du dibromure de 3-(6-{3-[4-(1H-imidazol-1-yl)butyl]-1H-imidazol-3-ium-1-yl} hexyl)-1-methyl-2-{(E)-[methyl(phenyl)hydrazono]methyl}-1 H-3,1-benzimidazol-3-ium

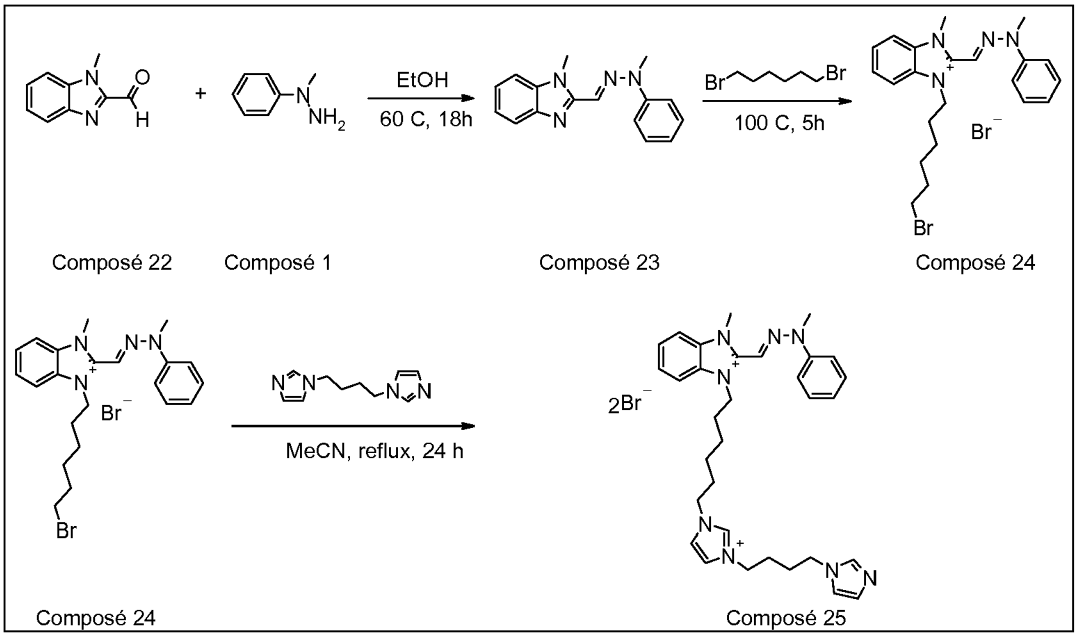


Composé 22 Composé 1 Composé 23 Composé 24

Composé 24 Composé 25

NB : le composé 22 est un composé commercial.

Etape 1

Dans un tricol de 250mL muni d'un thermomètre et surmonté d'un réfrigérant, on met sous agitation à 60°C, 2,4g (15mmoles) de 1-methyl-2-formylbenzimidazole [22] et 1,8g (15mmoles) de 1-methyl-1-phenylhydrazine [1] dans 50ml d'éthanol. Après 18 heures de réaction, le milieu réactionnel est ramené à température ambiante puis versé sur un bain de glace ; un précipité apparaît. Celui-ci est ensuite filtré, puis nettoyé avec de l'eau et enfin séché. 3,9 g du composé [23] sont obtenus.

Les spectres RMN et les spectres de Masse sont conformes au produit [23] attendu.

Etape 2

Dans un tricol de 250mL muni d'un thermomètre et surmonté d'un réfrigérant, on met sous agitation à 100°C, 3,9g (14,7 mmoles) d'intermédiaire [23] et 23ml (147 mmoles) de 1,6-dibromohexane. Après 5 heures de réaction, le milieu réactionnel est refroidi à température ambiante. Un précipité jaune se forme progressivement au cours du refroidissement. Celui-ci est ensuite filtré, nettoyé plusieurs fois à l'acétate d'éthyle puis séché. 7 g de composé [24] sont obtenus.

Les spectres RMN et les spectres de Masse sont conformes au produit [24] attendu.

Etape 3

Dans un tricol de 250mL muni d'un thermomètre et surmonté d'un réfrigérant, on met sous agitation à reflux, l'intermédiaire [24] (1,5 g, 3 mmoles), 5,7 g de 1,4-bis(1-imidazolyl)butane (30 mmoles) solubilisés dans 90 ml d'acétonitrile. Après 24 heures de reflux, le mélange réactionnel est refroidi puis concentré ; le résidu obtenu est ensuite purifié par chromatographie sur colonne d'alumine pour fournir 1,1 g de composé [25].

Les spectres RMN et les spectres de Masse sont conformes au produit [25] attendu.

**NB : Synthèse 1,4-bis(1-imidazolyl)butane**

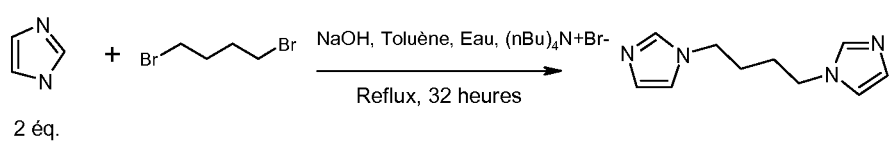


Dans un ballon surmonté d'un réfrigérant, on met sous agitation et à reflux pendant 32 heures, 35,2 g d'imidazole (0,52 mole), 54 g de 1,4-dibromobutane (0,26 mole), 100 g de soude (2,5 moles), 5,15 g de bromure de tétrabutylammonium (0,016 mole) dans 240 ml de toluène et 100 ml d'eau. Après refroidissement, un produit cristallise. Celui-ci est filtré et recristallisé dans 200 ml d'eau. Après séchage sous vide actif, 48 g d'une poudre beige correspondant au composé sont obtenus.

Les spectres RMN et les spectres de masse sont conformes à la structure du composé attendu.

**EXEMPLE 7**

**Synthèse du composé [27] :**

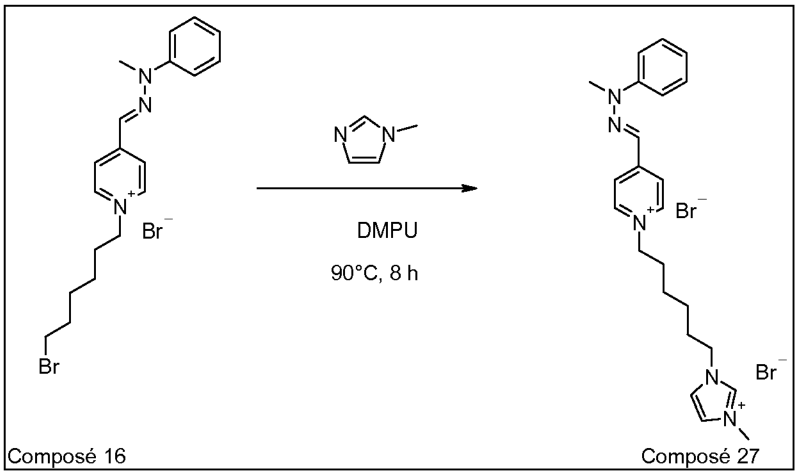


Composé 16 Composé 27

Dans un tricol de 250mL muni d'un thermomètre et surmonté d'un réfrigérant, on met sous agitation à 90˚C, l'intermédiaire [16] (2 g, 3,7 mmoles), le 1-methylimidazole (0,7 g, 3,7 mmoles) solubilisés dans 10 ml de DMPU. Après 8 heures à 90˚C, le mélange réactionnel est refroidi puis versé sur une solution d'acétate d'éthyle ; un précipité apparaît. Celui-ci est alors filtré, solubilisé avec un peu de méthanol et de nouveau précipité par ajout d'acétate d'éthyle. Le précipité est alors filtré, puis séché sous vide pour fournir 2,5 g de composé [27].

Les spectres RMN et les spectres de Masse sont conformes au produit [27] attendu.

## EXEMPLE 8

**Synthèse du composé [28] :** sel triple de dibromure et de 4-methylbenzenesulfonate de 1-(6-{3-[4-(3-methyl-1H-imidazol-3-ium-1-yl)butyl]-1H-imidazol-3-ium-1-yl}hexyl)-4-{(E)[methyl(phenyl)hydrazono]methyl}quinolinium.

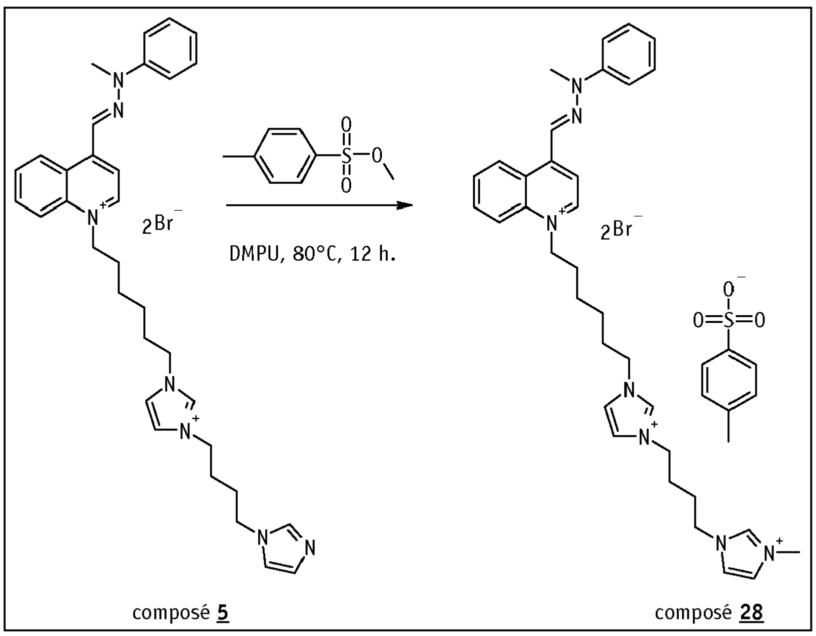


composé **5** composé **28**

Dans un tricol de 50mL muni d'un thermomètre et surmonté d'un réfrigérant, on met sous agitation à 80˚C, l'intermédiaire [5] (0.123 g, 0,2 mmole), le methyl *p*-toluene sulfonate (0,149 g, 0.8 mmole) solubilisés dans 1,5 ml de DMPU.

Après 12 heures à 80°C, le mélange réactionnel est refroidi puis versé sur une solution d'acétate d'éthyle ; une huile orangée visqueuse apparaît. Celle-ci est ensuite lavée 2 fois à l'acétate d'éthyle. L'huile orange terne obtenue est alors séchée sous vide pour fournir 0,097 g de composé [28].

Les spectres RMN et les spectres de Masse sont conformes au produit [28] attendu.

## EXEMPLE 9

**Synthèse du composé [29]** : sel triple de dibromure et de chlorure de 4-{(E)-[methyl(phenyl)hydrazono]methyl}-1-{6-[3-(4-{3-[3-(trimethylsilyl)propyl]-1H-imidazol-3-ium-1-yl}butyl)-1H-imidazol-3-ium-1-yl]hexyl}quinolinium

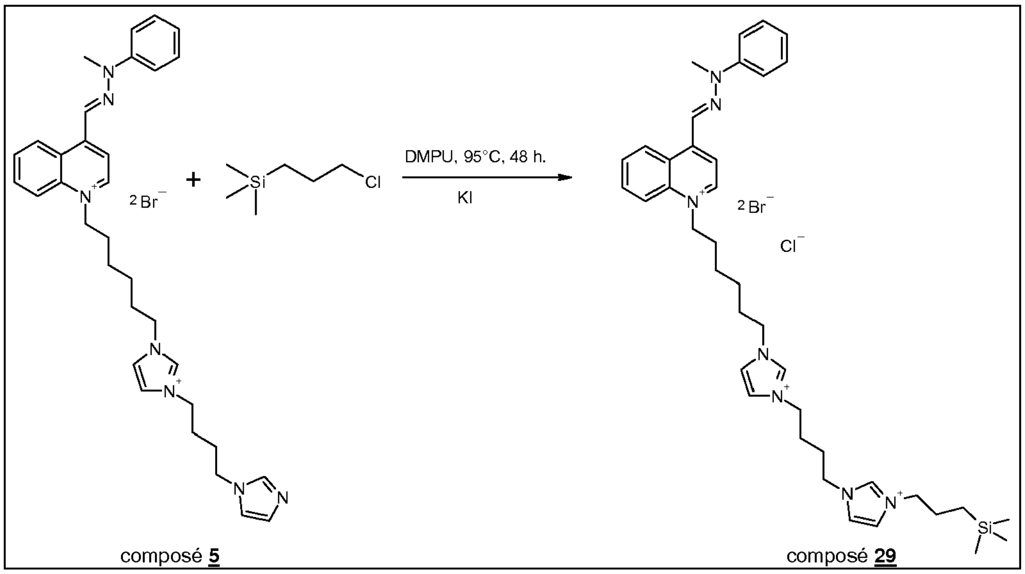


composé **5** composé **29**

Dans un tricol de 50mL muni d'un thermomètre et surmonté d'un réfrigérant, on met sous agitation à 95°C, l'intermédiaire [5] (0.123 g, 0,2 mmole), le (3-chloropropyl)(triméthyl)silane (0,121 g, 0.8 mmole) et une quantité catalytique d'iodure de potassium solubilisés dans 1,5 ml de DMPU.

Après 48 heures à 95°C, le mélange réactionnel est refroidi puis versé sur une solution d'acétate d'éthyle ; une huile orangée visqueuse apparaît. Celle-ci est ensuite purifiée par chromatographie sur colonne d'alumine pour fournir 0,081 g de composé [29].

Les spectres RMN et les spectres de Masse sont conformes au produit [29] attendu.

## EXEMPLE 10

**Synthèse du composé [31] :** sel double de bromure et de 4-méthylbenzènesulfonate de 1-[6-(3-methyl-1H-imidazol-3-ium-1-yl)hexyl]-2-{(E)-[methyl(phenyl)hydrazono]methyl} pyridinium.

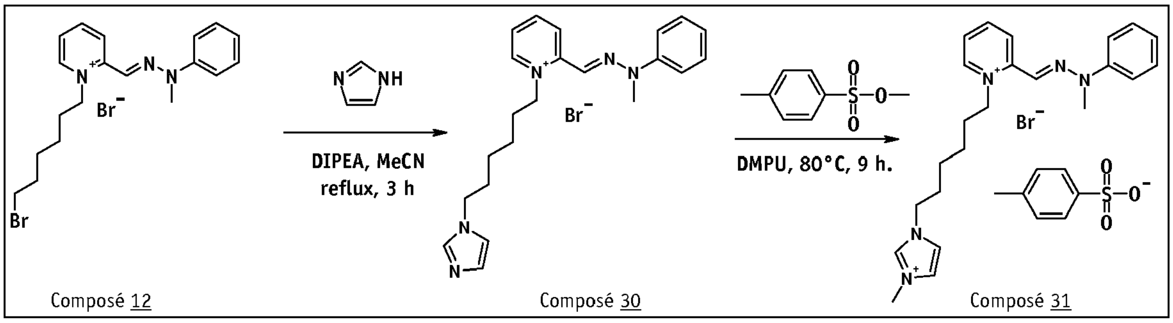


Composé 12 Composé 30 Composé 31

Etape 1

Dans un tricol de 25 mL muni d'un thermomètre et surmonté d'un réfrigérant, on met sous agitation, l'intermédiaire [12] (2,27 g, 5 mmoles), l'imidazole (3,40 g, 50 mmoles) dans 30 ml d'acétonitrile et 11,4 g de diisopropyléthylamine.

Après 3 heures de reflux, le milieu réactionnel est refroidi ; le solvant est évaporé et le résidu ainsi obtenu est ensuite chromatographié (THF / MeOH: 4:1). 2.0 g de produit correspondant à un mélange constitué du composé [30] et de quelques impuretés sont obtenus. Le produit est ensuite mis en suspension dans 80 ml d'acétate d'éthyle et 2 ml de méthanol. Après 12 heures d'agitation à température ambiante, la poudre jaune est filtrée puis séchée sous vide. 1,6 g d'une poudre jaune correspondant au composé [30] sont obtenus.

Les spectres RMN et les spectres de Masse sont conformes au produit [30] attendu.

Etape 2

Dans un tricol de 50mL muni d'un thermomètre et surmonté d'un réfrigérant, on met sous agitation à 80°C, l'intermédiaire [30] (0.089 g, 0,2 mmole), le methyl p-toluene sulfonate (0,149 g, 0.8 mmole) solubilisés dans 1,5 ml de DMPU.

Après 9 heures à 80°C, le mélange réactionnel est refroidi puis versé sur une solution d'acétate d'éthyle ; une huile jaune visqueuse apparaît. Celle-ci est ensuite lavée 2 fois à l'acétate d'éthyle. L'huile jaune obtenue est alors séchée sous vide pour fournir 0,061 g de composé [31].

Les spectres RMN et les spectres de Masse sont conformes au produit [31] attendu.

**EXEMPLE 11**

**Synthèse du composé [32] :** sel double de bromure et de chlorure de 2-{(E)-[methyl(phenyl)hydrazono] methyl}-1-(6-{3-[3-(trimethylsilyl)propyl]-1H-imidazol-3-ium-1-yl}hexyl)pyridinium.

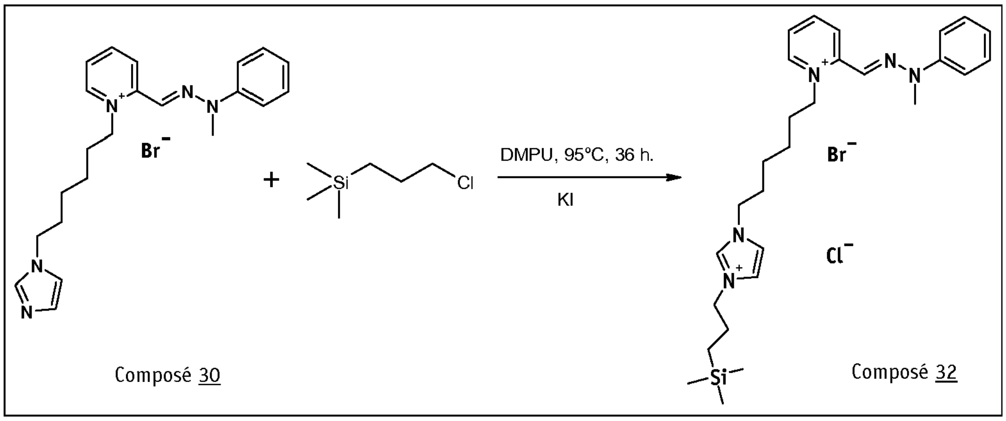


Composé 30 Composé 32

Dans un tricol de 50mL muni d'un thermomètre et surmonté d'un réfrigérant, on met sous agitation à 95°C, l'intermédiaire [30] (0.088 g, 0,2 mmole), le (3-chloropropyl)(trimethyl)silane (0,121 g, 0.8 mmole) et une quantité catalytique d'iodure de potassium solubilisés dans 1,5 ml de DMPU.

Après 36 heures à 95°C, le mélange réactionnel est refroidi puis versé sur une solution d'acétate d'éthyle ; un résidu solide apparaît. Celui-ci est filtré sous vide, lavé deux fois à l'acétate d'éthyle puis séché sous vide. 0,074 g d'une poudre jaune correspondant au composé [32] est obtenu.

Les spectres RMN et les spectres de Masse sont conformes au produit [32] attendu.

**EXEMPLE 12**

**Synthèse du composé [34]** : sel double de chlorure et de 4-methylbenzenesulfonate de 1-methyl-3-[6-(3-methyl-1H-imidazol-3-ium-1-yl)hexyl]-2-{(E)-[methyl(phenyl)hydrazono] methyl}-1H-3,1-benzimidazol-3-ium.

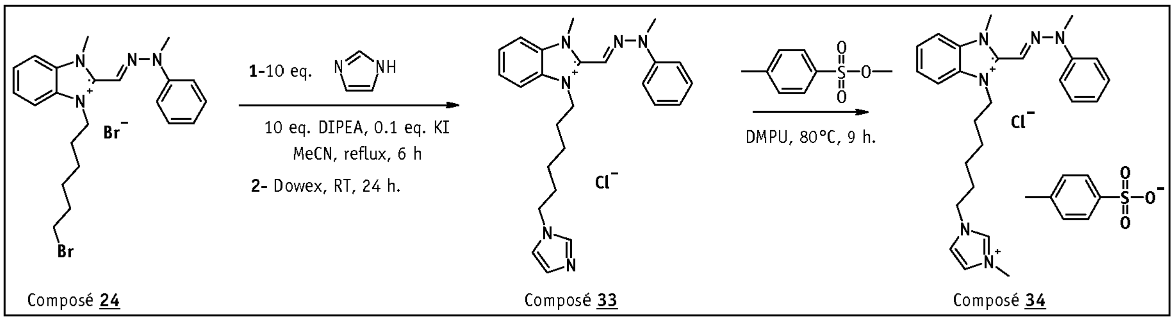


Composé **24** Composé **33** Composé **34**

Etape 1

Dans un tricol de 250mL muni d'un thermomètre et surmonté d'un réfrigérant, on met sous agitation, l'intermédiaire [24] (2,03 g, 4 mmoles), l'imidazole (2,72 g, 40 mmoles) dans 30 ml d'acétonitrile , 7 ml de diisopropyléthylamine et une quantité catalytique d'iodure de potassium (67 mg, 0.4 mmole).

Après 6 heures de reflux, le mélange réactionnel est refroidi puis versé sur 300 ml d'acétate d'éthyle ; le précipité obtenu est ensuite filtré, nettoyé avec de l'acétate d'éthyle et enfin séché sous vide. 1,8 g d'une poudre jaune sont obtenus.

La poudre obtenue est ensuite mise en solution dans 30 ml de méthanol en présence de 34 g de résine échange d'anion DOWEX. Le mélange est ensuite mis sous agitation à température ambiante pendant 24 heures. Après filtration, le milieu réactionnel est ensuite concentré sous vide. Le solide est ensuite purifié par chromatographie sur gel de silice pour fournir 0,86 g de composé [33] (solide jaune).

Les spectres RMN et les spectres de Masse sont conformes au produit [33] attendu.

Etape 2

Dans un tricol de 50mL muni d'un thermomètre et surmonté d'un réfrigérant, on met sous agitation à 80°C, l'intermédiaire [33] (0.099 g, 0,2 mmole), le methyl *p*-toluene sulfonate (0,149 g, 0.8 mmole) solubilisés dans 1,5 ml de DMPU.

Après 9 heures à 80°C, le mélange réactionnel est refroidi puis versé sur une solution d'acétate d'éthyle ; une huile jaune visqueuse apparaît. Celle-ci est ensuite lavée 2 fois à l'acétate d'éthyle. L'huile jaune obtenue est alors séchée sous vide pour fournir 0,065 g de composé [34].

Les spectres RMN et les spectres de Masse sont conformes au produit [34] attendu.

**EXEMPLE 13**

**Synthèse du composé [35]** : sel double de dichlorure de 1-methyl-2-{(E)-[methyl(phenyl)hydrazono]methyl}-3-(6-{3-[3-(trimethylsilyl)propyl]-1H-imidazol-3-ium-1-yl}hexyl)-1H-3,1-benzimidazol-3-ium

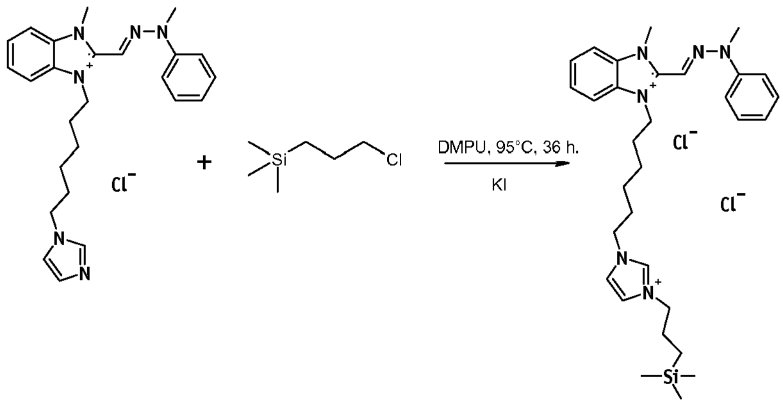


Composé **33** Composé **35**

Dans un tricol de 50mL muni d'un thermomètre et surmonté d'un réfrigérant, on met sous agitation, l'intermédiaire [33] (0.099 g, 0,2 mmole), le (3-chloropropyl)(trimethyl)silane (0,121 g, 0.8 mmole) et une quantité catalytique d'iodure de potassium solubilisés dans 1,5 ml de DMPU.

Après 36 heures à 95°C, le mélange réactionnel est refroidi puis versé sur une solution d'acétate d'éthyle ; un précipité jaune apparaît. Celui-ci est filtré sous vide, lavé deux fois à l'acétate d'éthyle puis séché sous vide. 0,087 g d'une poudre jaune correspondant au composé [35] est obtenu.

Les spectres RMN et les spectres de Masse sont conformes au produit [35] attendu.

## EXEMPLE 14

### Exemples de teintures

On a préparé les compositions tinctoriales dans les proportions suivantes:

**Solution 1**

| | |
|---|---|
| Alkyl (C8/C10 50/50) polyglucoside (2) en solution aqueuse à 60% | 120 g |
| Ethanol | 200 g |
| Polyéthylène glycol(8 OE) 400 | 60 g |
| Alcool benzylique | 40 g |
| Eau déminéralisée | qsp 1000g |

**Solution 2 :** TAMPON pH 9,5

| | |
|---|---|
| Chlorure d'ammonium ($NH_4Cl$) | 54 g |
| Ammoniaque en solution aqueuse à 20% | qsp pH=9,5 (env. 40 ml) |
| Eau déminéralisée | qsp 1000 ml |

**Solution 3 :** TAMPON pH 7

| | |
|---|---|
| $KH_2PO_4$ | 0,026mol/l |
| $Na_2PO_4$ | 0,041 mol/l |
| Eau déminéralisée | qsp 500ml |

Les compositions de coloration sont obtenues en dissolvant le colorant indiqué ci dessous ($5x10^{-3}$mol/l) dans la solution 1 puis en ajoutant un volume équivalent de solution 2 ou 3 (pH 7 ou 9,5).

Chaque composition est appliquée sur des cheveux naturels à 90% de blancs, (1g de mèche pour 6g de solution).

Après 30 minutes à température ambiante, les mèches sont rincées, lavées avec un shampooing standard, rincées à nouveau puis séchées.

Les résultats sont dans le tableau ci-dessous :

| | **pH7** | **pH 9,5** |
|---|---|---|
| 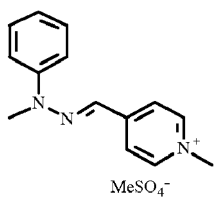 **Composé 1** : Basic Yellow 87 (non conforme à l'invention) | Jaune chromatique | Jaune chromatique |
| **Composé 5** | orange chromatique intense | orange chromatique intense |
| **Composé 9** | Jaune orangé chromatique intense | Jaune orangé chromatique intense |
| **Composé 13** | Jaune | Jaune |
| **Composé 17** | Jaune chromatique intense | Jaune chromatique Intense |
| **Composé 21** | Jaune pâle | Jaune pâle |
| **Composé 25** | Jaune | Jaune |
| **Composé 27** | Jaune chromatique intense | Jaune chromatique intense |
| **Composé 28** | Orange chromatique intense | Orange chromatique intense |
| **Composé 29** | Orange chromatique intense | Orange chromatique intense |
| **Composé 31** | Jaune | Jaune |
| **Composé 32** | Jaune pâle | Jaune pâle |
| **Composé 34** | Jaune pâle | Jaune pâle |
| **Composé 35** | Jaune pâle | Jaune pâle |

**Essais comparatifs**

La couleur des mèches colorées par le composé (Basic Yellow 87 non conforme à l'invention) et le composé 27 a ensuite été évaluée au moyen d'un spectrophotomètre CM 2002 MINOLTA ®, (Illuminant D65).

On calcule la chromaticité qui correspond à la formule suivante:

$$C^* = [\, a^{*2} + b^{*2} \,]^{1/2}$$

Plus C* est élevée, plus la coloration obtenue est chromatique.

On calcule également la montée du colorant qui correspond à la formule suivante :

$$DE^* = [ (L^*_0 - L^*_1)^2 + (a^*_0 - a^*_1)^2 + (b^*_0 - b^*_1)^2 ]^{1/2}$$

Où $L^*_0$, $a^*_0$ et $b^*_0$ correspond aux coefficients obtenus avec les mèches non colorées, $L^*_1$, $a^*_1$ et $b^*_1$ correspond aux coefficients obtenus avec les mèches colorées.

Plus la valeur de DE* est importante, meilleure est la montée du colorant.

Les résultats suivants ont été obtenus :

| | pH | C* | DE * |
|---|---|---|---|
| **Composé I** | 7 | 40,25 | 30,5 |
| **Composé 27** | 7 | 59 | 45,5 |
| | | | |
| **Composé I** | 9,5 | 35,6 | 26,05 |
| **Composé 27** | 9,5 | 48,4 | 35,35 |

La coloration obtenue à partir du composé **27** est significativement plus chromatique et la montée est plus importante que dans le cas de la coloration obtenue à partir du composé I, pour les deux valeurs de pH.

Par la suite, ces mèches ont été sont soumises à un test de résistance aux shampooings qui consiste à effectuer 10 shampoings (lavage, rinçage) et à mesurer la couleur après ces 10 shampooings.

La couleur a été évaluée par la mesure du L*a*b* telle que définie précédemment.

Les résultats suivants ont été obtenus :

| | Après 10 shampooings (% de perte de la couleur) |
|---|---|
| **Composé I** | 20-30 |
| **Composé 27** | 0-10 |

## Revendications

1. Composés polycationiques de type hydrazone de formule (I) suivante, leurs formes tautomères ainsi que leurs sels d'addition avec un acide et leurs solvates :

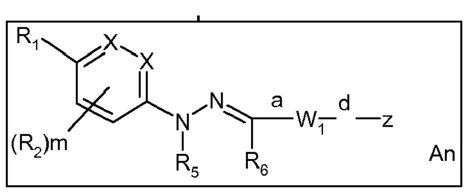

(I)

dans laquelle :

$W_1$ représente un radical hétéroaromatique de formules (1) à (8) suivantes :

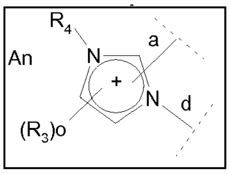
(1)

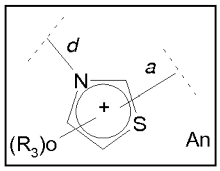
(2)

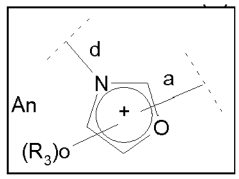
(3)

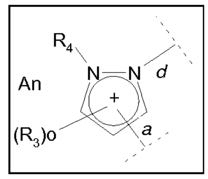
(4)

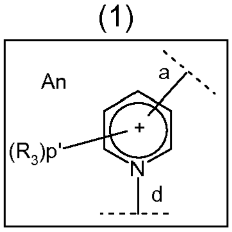
(5)

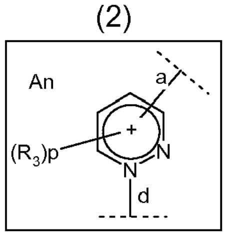
(6)

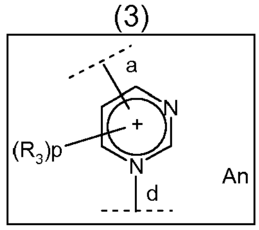
(7)

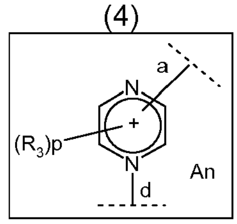
(8)

Où:

* le radical $R_1$ représente:

- un hydrogène ;
- un radical alkyle en $C_1$-$C_{20}$, éventuellement substitué, éventuellement interrompu par un ou plusieurs hétéroatomes et/ou par un ou plusieurs groupements comprenant au moins un hétéroatome, de préférence choisis parmi l'oxygène, l'azote, le soufre, CO, SO, $SO_2$ ou leurs combinaisons ; le radical alkyle ne comportant pas de fonction nitro, nitroso, peroxo ou diazo ;
- un radical alkylcarbonyle (R-CO-) dans lequel R représente un radical alkyle en $C_1$-$C_4$ ;
- un radical alkylsulfonyle ($RSO_2$-) dans lequel R représente un radical alkyle en $C_1$-$C_4$ ;
- un radical arylsulfonyle ($R'SO_2$-) dans lequel R' représente un radical phényle ou benzyle éventuellement substitué ;
- un radical (di-)(alkyl)aminosulfonyle ($(R)_2N$-$SO_2$-) dans lequel les radicaux R indépendamment représentent un hydrogène, un radical alkyle en $C_1$-$C_4$ ;
- un radical (di-)(alkyl) aminocarbonyle $(R)_2N$-CO-) dans lequel les radicaux R indépendamment représentent un hydrogène, un radical alkyle en $C_1$-$C_4$ ;
- un atome d'halogène choisi de préférence parmi le brome, le chlore ou le fluor ;
- un groupement hydroxyle ;
- un groupement alcoxy en $C_1$-$C_4$ ;
- un groupement (poly)hydroxyalcoxy en $C_2$-$C_4$;
- un groupement hydroxycarbonyle ;
- un groupement alcoxycarbonyle (RO-CO-) dans lequel R représente un radical alkyle en $C_1$-$C_4$ ;
- un radical alkylcarbonyloxy (RCO-O-) dans lequel R représente un radical alkyle en $C_1$-$C_4$ ;
- un groupement aryloxy éventuellement substitué ;
- un groupement $NR_7R_8$ dans lequel $R_7$ et $R_8$ représentent indépendamment l'un de l'autre :

  • un atome d'hydrogène;
  • un radical alkyle en $C_1$-$C_4$, éventuellement porteur d'au moins un groupement hydroxyle ou alcoxy en $C_1$-$C_2$, lesdits radicaux alkyle pouvant éventuellement former avec l'atome d'azote auquel ils sont rattachés, un hétérocycle comprenant de 5 à 7 chaînons, saturé ou insaturé, éventuellement substitué, éventuellement aromatique, comprenant éventuellement un autre hétéroatome identique ou différent de l'azote ;
  • un radical phényle ; un radical aminophényle ; un radical 4-N,N-diéthylaminophényle ; un radical méthoxyphényle ;

- un groupement alkylcarbonylamino (RCO-NR'-) dans lequel le radical R représente un radical alkyle en $C_1$-$C_4$ et le radical R' représente un atome d'hydrogène ou un radical alkyle en $C_1$-$C_4$;
- un groupement uréido ($N(R)_2$-CO-NR'-) dans lequel les radicaux R et R',indépendamment les uns des autres, représentent un atome d'hydrogène, un radical alkyle en $C_1$-$C_4$ ;
- un groupement alkylthio (R-S-) dans lequel le groupement R représente un radical alkyle en $C_1$- $C_4$ ;
- un groupement alkylsulfonylamino ($RSO_2$-NR'-) dans lequel le radical R', représente un atome d'hydrogène ou un radical alkyle en $C_1$-$C_4$, et le radical R représente un radical alkyle en $C_1$-$C_4$ ;
- un groupement cyano ;
- un groupement trifluorométhyle($CF_3$);

* Les radicaux $R_2$ et $R_3$, indépendamment les uns des autres, identiques ou différents représentent :

- un atome d'halogène;
- un radical alkyle en $C_1$-$C_{16}$ éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, $SO_2$ ou leurs combinaisons ; le radical alkyle ne comportant pas de fonction nitro, nitroso, peroxo ou diazo ;
- un radical hydroxyle ;
- un radical alcoxy en $C_1$-$C_4$ ;
- un groupement (poly)hydroxyalcoxy en $C_2$-$C_4$ ;
- un radical hydroxycarbonyle ;
- un radical alcoxycarbonyle (RO-CO-) dans lequel R représente un radical alkyle en $C_1$-$C_4$ ;
- un radical alkylcarbonyloxy (RCO-O-) dans lequel R représente un radical alkyle en $C_1$-$C_4$ ;
- un radical aryloxy éventuellement substitué ;
- un radical arylamino éventuellement substitué ;
- un radical amino éventuellement substitué par un ou deux radicaux alkyle en $C_1$-$C_4$, identiques ou différents, éventuellement porteurs d'au moins un groupement hydroxyle ou alcoxy en $C_1$-$C_2$, lesdits radicaux alkyle pouvant éventuellement former avec l'atome d'azote auquel ils sont rattachés, un hétérocycle comprenant de 5 à 7 chaînons, saturé ou insaturé, éventuellement substitué, éventuellement aromatique, comprenant éventuellement un autre hétéroatome identique ou différent de l'azote ;
- un radical alkylcarbonylamino (RCO-NR'-) dans lequel le radical R représente un radical alkyle en $C_1$-$C_4$ et R' représente un hydrogène, un radical alkyle en $C_1$-$C_4$ ;
- un groupement (di-)(alkyl)aminocarbonyle ($(R)_2N$-CO-) dans lequel les radicaux R, indépendamment l'un de l'autre représentent un hydrogène, un radical alkyle en $C_1$-$C_4$ ;
- un radical uréido ($N(R)_2$-CO-NR'-) dans lequel les radicaux R, indépendamment les uns des autres, représentent un radical alkyle en $C_1$-$C_4$ et R' représente un hydrogène, un radical alkyle en $C_1$-$C_4$ ;
- un radical (di-)(alkyl) aminosulfonyle ($(R)_2N$-$SO_2$-) dans lequel le radical R représente un atome d'hydrogène, un radical alkyle en $C_1$-$C_4$ ;
- un radical alkylthio (R-S-) dans lequel le radical R représente un radical alkyle en $C_1$-$C_4$ ;
- un radical alkylsulfonylamino ($RSO_2$-NR'-) dans lequel le radical R représente un radical alkyle en $C_1$-$C_4$, et le radical R' représente un atome d'hydrogène, un radical alkyle en $C_1$-$C_4$ ;
- un radical alkylsulfonyle ($RSO_2$-) dans lequel le radical R représente un radical alkyle en $C_1$-$C_4$ ;
- un radical cyano (-CN) ;
- un radical trifluorométhyle (-$CF_3$) ;

* Deux radicaux $R_2$ adjacents peuvent former entre eux et avec les atomes de carbone auxquels ils sont reliés, un radical (hétéro)cyclique aromatique ou non, à 5 ou 6 chaînons, substitué ou non ;
* L'un des radicaux $R_7$ ou $R_8$ peut former avec l'atome d'azote auquel il est rattaché et avec un radical $R_2$ situé en ortho du groupement $NR_7R_8$, un hétérocycle saturé ou insaturé à 5 ou 6 chaînons substitué ou non
* Les radicaux $R_7$ et $R_8$ peuvent former avec l'atome d'azote auquel ils sont rattachés et chacun avec un radical $R_2$ situé en ortho du groupement $NR_7R_8$, un hétérocycle saturé ou insaturé à 5 ou 6 chaînons substitué ou non
* m est un entier compris entre 0 et 4 lorsque m est inférieur à 4, le ou les atomes de carbone non substitués porte(nt) un atome d'hydrogène;
* X indépendamment l'un de l'autre représente N, $CR_2$;
* o est un entier compris entre 0 et 2 ; lorsque o est inférieur à 2, le ou les atomes de carbone non substitués portent un atome d'hydrogène ;
* Deux radicaux $R_3$ adjacents peuvent former entre eux et avec les atomes de carbone auxquels ils sont rattachés, un cycle aromatique à 6 chaînons, substitué ou non;
* Les radicaux $R_4$, identiques ou différents, représentent :

- un radical alkyle en $C_1$-$C_{20}$, éventuellement substitué, éventuellement interrompu par un ou plusieurs hétéroatomes et/ou par un ou plusieurs groupements comprenant au moins un hétéroatome, de préférence choisis parmi l'oxygène, le soufre, CO, SO, $SO_2$ ou leurs combinaisons; le radical alkyle ne

comportant pas de fonction nitro, nitroso, peroxo ou diazo ;
- un radical triméthylsilylalkyle en $C_1$-$C_4$ ;
- un radical phényle éventuellement substitué;
- un radical benzyle éventuellement substitué;

* Le radical $R_5$ représente :

- un hydrogène ;
- un radical alkyle en $C_1$-$C_{20}$, éventuellement substitué, éventuellement interrompu par un ou plusieurs hétéroatomes et/ou par un ou plusieurs groupements comprenant au moins un hétéroatome, de préférence choisis parmi l'oxygène, l'azote, le soufre, CO, SO, $SO_2$ ou leurs combinaisons ; le radical alkyle ne comportant pas de fonction nitro, nitroso, peroxo ou diazo ;
- un radical phényle éventuellement substitué ;
- un radical benzyle éventuellement substitué ;
- un radical alkylcarbonyle (R-CO-) dans lequel R représente un radical alkyle en $C_1$-$C_4$;
- un radical alkylsulfonyle ($RSO_2$-) dans lequel R représente un radical alkyle en $C_1$-$C_4$;
- un radical arylsulfonyle ($R'SO_2$-) dans lequel R' représente un radical phényle ou benzyle éventuellement substitué ;
- un radical (di-)(alkyl)aminosulfonyle ($(R)_2N$-$SO_2$-) dans lequel les radicaux R indépendamment représentent un hydrogène, un radical alkyle en $C_1$-$C_4$;
- un radical (di-)(alkyl) aminocarbonyle $(R)_2N$-CO-) dans lequel les radicaux R indépendamment représentent un hydrogène, un radical alkyle en $C_1$-$C_4$;

* $R_5$ peut former avec un radical $R_2$ situé en ortho du groupement $NR_5$ et avec l'atome d'azote substitué par $R_5$, un hétérocycle saturé ou insaturé à 5 ou 6 chaînons substitué ou non ;
* Le radical $R_6$, représente :

- un atome d'hydrogène ;
- un radical alkyle en $C_1$-$C_{16}$, éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, $SO_2$, ou leurs combinaisons ; le radical alkyle ne comportant pas de fonction nitro, nitroso, peroxo ou diazo ;
- un radical amino éventuellement substitué par un ou deux radicaux alkyle en $C_1$-$C_4$, identiques ou différents, éventuellement porteurs d'au moins un groupement hydroxyle ou alcoxy en $C_1$-$C_2$, lesdits radicaux alkyle pouvant éventuellement former avec l'atome d'azote auquel ils sont rattachés, un hétérocycle à 5 ou 6 chaînons saturé ou insaturé ou aromatique éventuellement substitué, comprenant éventuellement au moins un autre hétéroatome identique ou différent de l'azote ;
- un radical alkylcarbonylamino (RCO-NR'-) dans lequel le radical R représente un radical alkyle en $C_1$-$C_4$ et R' représente un hydrogène, un radical alkyle en $C_1$-$C_4$;
- un radical uréido ($N(R)_2$-CO-NR'-) dans lequel les radicaux R, indépendamment les uns des autres, représentent un radical alkyle en $C_1$-$C_4$ et R' représente un hydrogène, un radical alkyle en $C_1$-$C_4$;
- un radical alkylsulfonylamino ($RSO_2$-NR'-) dans lequel le radical R représente un radical alkyle en $C_1$-$C_4$ et R' représente un hydrogène, un radical alkyle en $C_1$-$C_4$ ;
- un radical hydroxycarbonyle (HOOC-) ;
- un radical alcoxycarbonyle en $C_1$-$C_4$ (RO-CO-) ;
- un radical phényle éventuellement substitué ;
- un radical benzyle éventuellement substitué ;

* p est un entier compris entre 0 et 3 ; p' est un entier compris entre 0 et 4 ; lorsque p est inférieur à 3 ou lorsque p' est inférieur à 4, le ou les atomes de carbone non substitués porte(nt) un atome d'hydrogène ;
* La liaison a relie le radical hétéroaromatique $W_1$ à l'atome de carbone du groupement $CR_6$ ;
* Z représente un radical alkylène en $C_2$-$C_{20}$ comprenant au moins un atome d'azote quaternisé ;
* la liaison d relie le(s) groupement(s) cationique(s) Z à un atome d'azote du radical hétéroaromatique $W_1$;
* l'électroneutralité des composés de formule (I) étant assurée par un ou plusieurs anions ou mélange d'anions An cosmétiquement acceptables, identiques ou non.

2. Composés polycationiques selon la revendication précédente, **caractérisés en ce que** $R_1$ est choisi parmi :

- un hydrogène ;
- un radical alkyle en $C_1$-$C_4$ éventuellement substitué par un groupement hydroxy, alcoxy en $C_1$-$C_2$
- un atome de chlore
- un groupement hydroxyle ;
- un groupement alcoxy en $C_1$-$C_4$ ;
- un groupement hydroxyalcoxy en $C_2$-$C_4$ ;
- un groupement hydroxycarbonyle ;
- un groupement alcoxycarbonyle (RO-CO-) dans lequel R représente un radical alkyle en $C_1$-$C_2$ ;
- un groupement aryloxy non substitué ;
- un groupement $NR_7R_8$ dans lequel $R_7$ et $R_8$ représentent indépendamment l'un de l'autre :

  • un atome d'hydrogène ;
  • un radical alkyle en $C_1$-$C_4$, éventuellement porteur d'au moins un groupement hydroxyle ou alcoxy en $C_1$-$C_2$, lesdits radicaux alkyle pouvant éventuellement former avec l'atome d'azote auquel ils sont rattachés, un hétérocycle comprenant de 5 à 7 chaînons, saturé ou insaturé, éventuellement substitué, éventuellement aromatique, comprenant éventuellement un autre hétéroatome identique ou différent de l'azote ;
  • un radical phényle ; un radical 4-N,N-diéthylaminophényle ; un radical méthoxyphényle;

- un groupement alkylcarbonylamino (RCO-NR'-) dans lequel le radical R représente un radical alkyle en $C_1$-$C_4$ et le radical R' représente un atome d'hydrogène ou un radical alkyle en $C_1$-$C_4$ ;
- un groupement alkylsulfonylamino ($RSO_2$-NR'-) dans lequel le radical R', représente un atome d'hydrogène ou un radical alkyle en $C_1$-$C_2$, et le radical R représente un radical alkyle en $C_1$-$C_2$.

**3.** Composés polycationiques selon l'une quelconque des revendications précédentes, **caractérisés en ce que** $R_2$, $R_3$, indépendamment les uns des autres, sont choisis parmi :

- un atome de chlore
- un radical alkyle en $C_1$-$C_8$ éventuellement substitué par un ou plusieurs groupements, identiques ou non, choisis parmi les radicaux hydroxy, alcoxy en $C_1$-$C_2$, amino, thio, ((di-)alkyl)amino en $C_1$-$C_2$, alkylsulfonylamino en $C_1$-$C_2$, alkylsulfonyle en $C_1$-$C_2$
- un radical hydroxyle
- un radical alcoxy en $C_1$-$C_4$;
- un radical alcoxy($C_2$-$C_4$)carbonyle
- un radical aryloxy éventuellement substitué
- un radical amino éventuellement substitué :

  • par un ou deux radicaux alkyle en $C_1$-$C_4$, identiques ou différents, éventuellement porteurs d'un groupement hydroxyle ou alcoxy en $C_1$-$C_2$, lesdits radicaux alkyle pouvant éventuellement former avec l'atome d'azote auquel ils sont rattachés, un hétérocycle à 5 ou 7 chaînons, saturé ou insaturé, éventuellement substitué ;
  ■ par un ou deux radicaux phényle, aminophényle ou méthoxyphényle

- un radical alkyl($C_1$-$C_4$)carbonylamino dans lequel la fonction amino est substituée ou non par un radical alkyle en $C_1$-$C_4$
- un radical hydroxycarbonyle
- un radical aminocarbonyle
- un radical aminosulfonyle, (di)alkyl($C_1$-$C_4$)aminosulfonyle
- un radical alkyl($C_1$-$C_2$)sulfonylamino
- un radical alkyl($C_1$-$C_2$)sulfonyle
- un radical alkyl($C_1$-$C_4$)thio
- un radical phényle
- un radical trifluorométhyle
- un radical thio.

**4.** Composés polycationiques selon l'une quelconque des revendications précédentes, **caractérisés en ce que** deux radicaux $R_2$ adjacents forment entre eux et avec les atomes de carbone auxquels ils sont reliés, un radical cyclique, éventuellement aromatique comprenant 5 ou 6 chaînons, substitué ou non.

**5.** Composés selon l'une des revendications précédentes, **caractérisés en ce que** deux radicaux $R_3$ adjacents forment entre eux et avec les atomes de carbone auxquels ils sont rattachés, un cycle aromatique à 6 chaînons, substitué ou non.

**6.** Composés selon l'une des revendications précédentes, **caractérisés en ce que** le radical $R_4$ représente :

- un radical alkyle en $C_1$-$C_8$, de préférence en $C_1$-$C_8$, éventuellement substitué par un radical choisi parmi hydroxy, alcoxy en $C_1$-$C_4$, amino éventuellement substitué par un ou deux radicaux alkyle en $C_1$-$C_2$
- un radical triméthylsilylalkyle en $C_1$-$C_4$
- un radical phényle éventuellement substitué par un ou deux groupements choisis parmi le chlore, un radical hydroxy, un radical amino,
- un radical benzyle éventuellement substitué par un ou deux groupements choisis parmi le chlore, un radical hydroxy, un radical amino.

**7.** Composés polycationiques selon l'une quelconque des revendications précédentes, **caractérisés en ce que** $R_5$ représente:

- un hydrogène
- un radical alkyle en $C_1$-$C_6$, éventuellement substitué par au moins un groupement hydroxy, par au moins un groupement alcoxy en $C_1$-$C_2$, par au moins un groupement un groupement hydroxycarbonyle
- un radical phényle éventuellement substitué par au moins un atome d'halogène tels que le de chlore, le brome, l'iode, le de fluor ; par au moins un groupement hydroxyle et/ou amino
- un radical benzyle éventuellement substitué par un ou plusieurs hydroxy et/ou amino
- un radical alkyl($C_1$-$C_4$)carbonyle
- un radical alkyl($C_1$-$C_4$)sulfonyle.

**8.** Composés polycationiques selon l'une quelconque des revendications précédentes, **caractérisés en ce que** $R_5$ forme avec un radical $R_2$ situé en ortho du groupement $R_5$ et avec l'atome d'azote substitué par $R_5$, un hétérocycle saturé ou insaturé, à 5 ou 6 chaînons, substitué ou non.

**9.** Composés polycationiques selon l'une quelconque des revendications précédentes, **caractérisés en ce que** $R_6$ représente :

- un hydrogène,
- un radical méthyle, éthyle, 2-hydroxyéthyle,
- un radical amino,
- un radical hydroxycarbonyle
- un radical méthoxycarbonyle
- un groupement méthylcarbonylamino ($CH_3CO$-NH-);
- un groupement méthylsulfonylamino ($CH_3SO_2$-NH-);
- un radical phényle éventuellement substitué par un groupement hydroxy et/ou amino ou leur combinaison.
- un radical benzyle éventuellement substitué par un groupement hydroxy et/ou amino ou leur combinaison.

**10.** Composés polycationiques selon l'une quelconque des revendications précédentes, **caractérisés en ce que** Z représente Z cationique, celui-ci représente avantageusement un radical alkylène en $C_2$-$C_{20}$ :

1- interrompu par un ou plusieurs groupements, identiques ou non, correspondant aux formules suivantes :

| | |
|---|---|
| 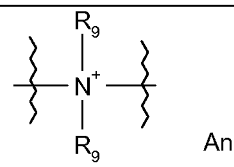 (a) | 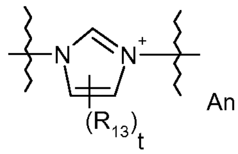 (b) |
| 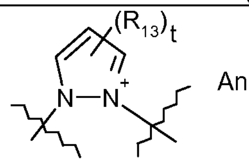 (c) | 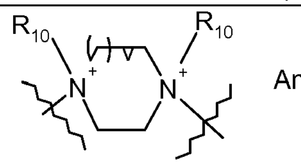 (d) |
| 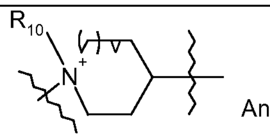 (e) | 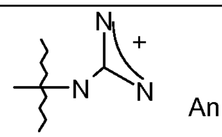 (f) |

Dans lesquelles :

- $R_9$ et $R_{10}$ indépendamment l'un de l'autre, représentent un radical alkyle en $C_1$-$C_8$ ; un radical monohydroxyalkyle en $C_1$-$C_6$ ; un radical polyhydroxyalkyle en $C_2$-$C_6$ ; un radical alcoxy($C_1$-$C_6$)alkyle en $C_1$-$C_6$ ; un radical aryle tel que phényle éventuellement substitué; un radical arylalkyle tel que benzyle éventuellement substitué; un radical aminoalkyle en $C_1$-$C_6$ dont l'amine est substituée par un ou deux radicaux alkyle $C_1$-$C_4$ identiques ou différents ; un radical alkyl($C_1$-$C_6$)sulfonyle ;
- deux radicaux $R_9$ peuvent former ensemble, avec l'atome d'azote auxquels ils sont rattachés, un cycle saturé ou insaturé, éventuellement substitué, à 5, 6 ou 7 chaînons ;
- $R_{13}$, identiques ou différents, représentent un atome d'hydrogène, un atome d'halogène choisi parmi le brome, le chlore ou le fluor, un radical alkyle en $C_1$-$C_6$, un radical monohydroxyalkyle en $C_1$-$C_6$, un radical polyhydroxyalkyle en $C_2$-$C_6$, un radical alcoxy en $C_1$-$C_6$, un radical (di-) alkylamino en $C_1$-$C_4$, un radical hydroxycarbonyle, un radical alkylcarbonyle en $C_1$-$C_6$, un radical thioalkyle en $C_1$-$C_6$, un radical alkyl($C_1$-$C_6$) thio, un radical alkyl($C_1$-$C_6$)sulfonyle, un radical benzyle éventuellement substitué, un radical phényle éventuellement substitué par un ou plusieurs radicaux choisis parmi les radicaux méthyle, hydroxyle, amino, méthoxy ;
- An est un anion ou un mélange d'anions, organiques ou minéraux ;
- t est un nombre entier compris entre 0 et 3 ; si t < 3, alors les atomes de carbone non substitués portent un atome d'hydrogène ;
- v est un entier égal à 1 ou 2 de préférence égal à 1 ;

2- et éventuellement interrompu par un ou plusieurs hétéroatomes ou groupements comprenant au moins un hétéroatome ou leurs combinaisons, comme par exemple l'oxygène, l'azote, le soufre, un groupement -CO-, un groupement $-SO_2-$ ; à la condition qu'il n'y ait pas de groupe ou liaison azo, nitro, nitroso, peroxo dans le groupement Z ;

3- et éventuellement substitué par un ou plusieurs radicaux choisis parmi les radicaux hydroxyle, alcoxy en $C_1$-$C_2$, (poly)-hydroxyalcoxy en $C_2$-$C_4$, amino substitué par un ou deux groupements alkyle linéaires en $C_1$-$C_2$ éventuellement porteurs d'au moins un groupement hydroxyle, trialkyl($C_1$-$C_4$)silyle, imidazole.

**11.** Composés polycationiques selon l'une quelconque des revendications précédentes, **caractérisés en ce que** X représente $CR_2$.

**12.** Composés polycationiques selon l'une quelconque des revendications précédentes, **caractérisés en ce qu'**ils correspondent aux formules suivantes, leurs formes tautomères ainsi que leurs sels d'addition avec un acide ou leurs solvates :

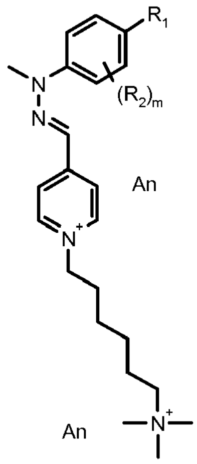

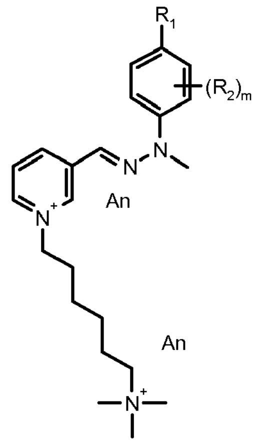

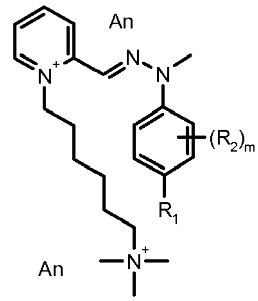

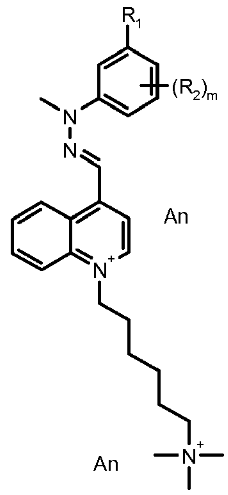

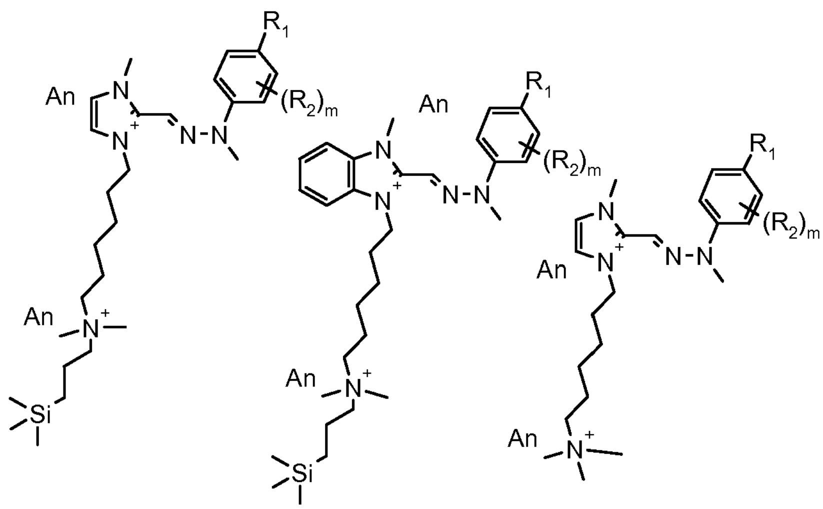

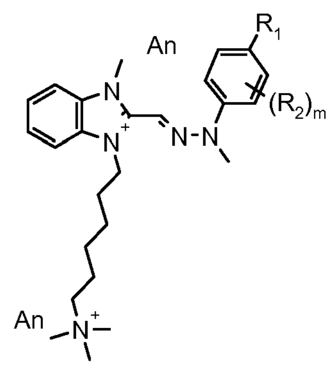

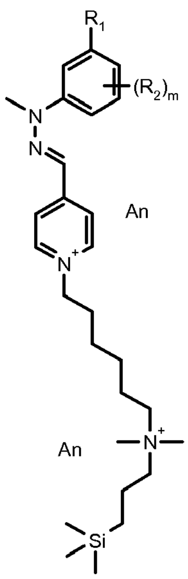

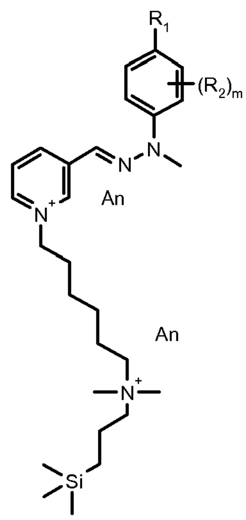

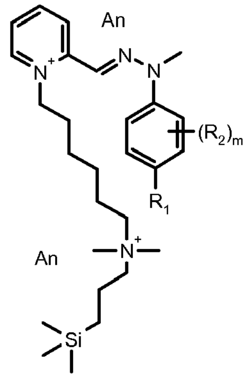

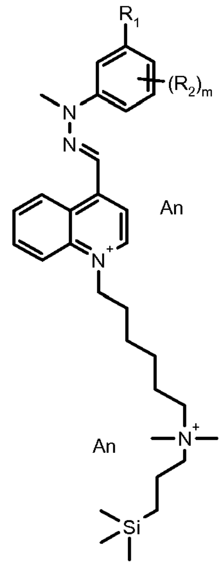

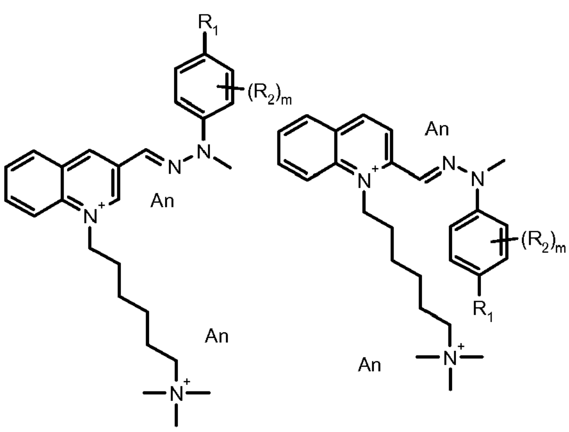

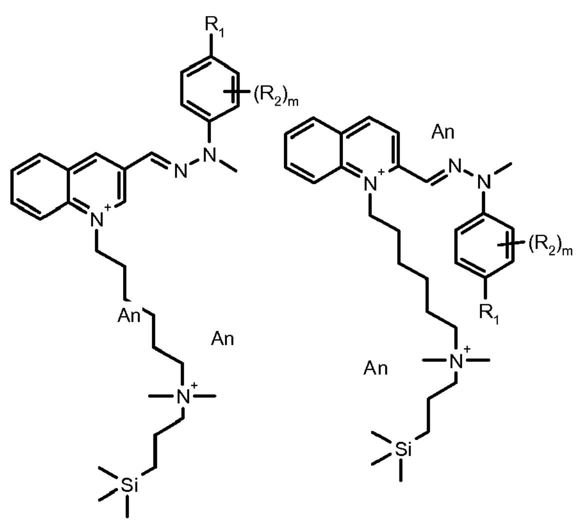

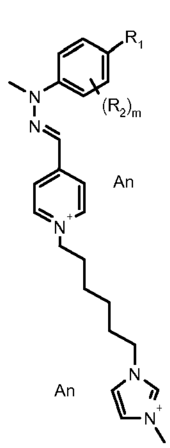

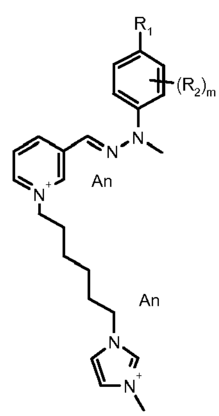

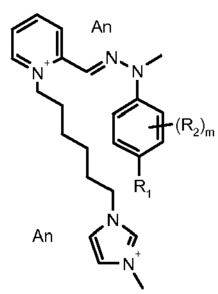

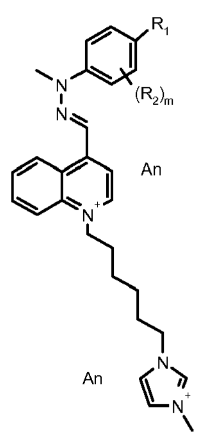

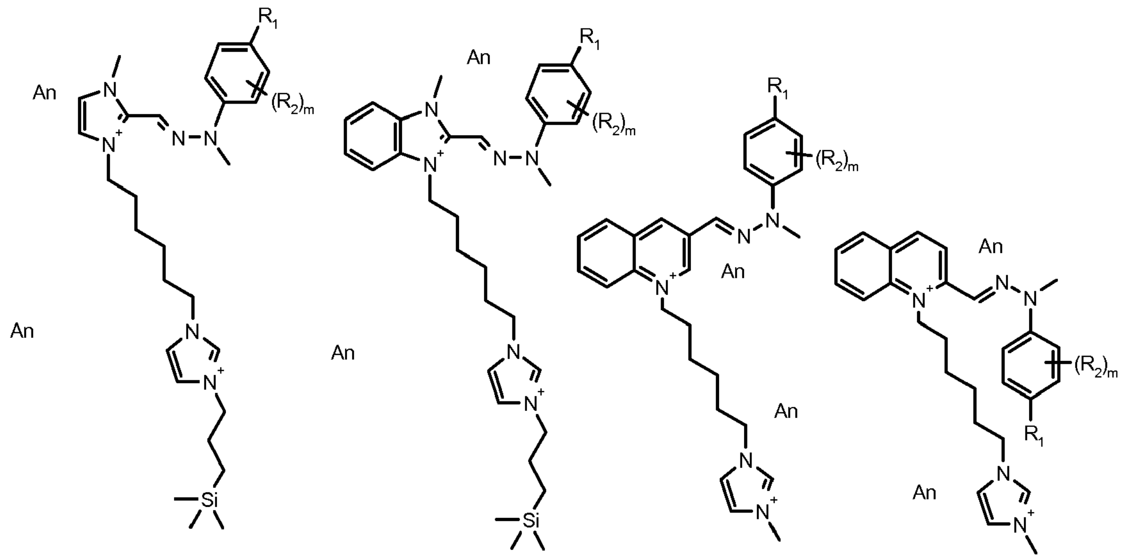

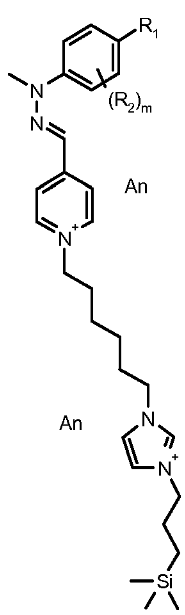

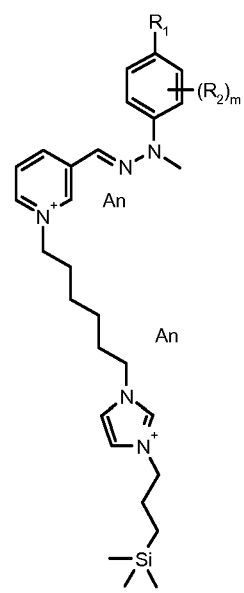

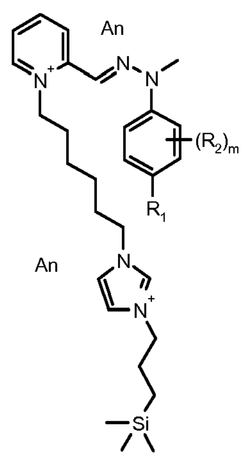

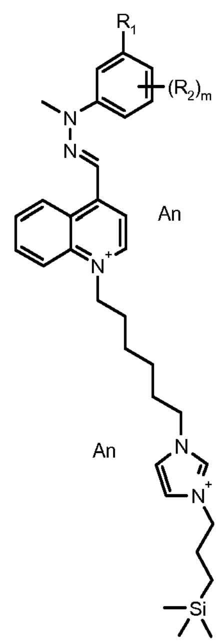

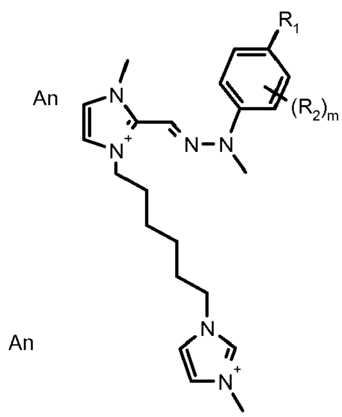

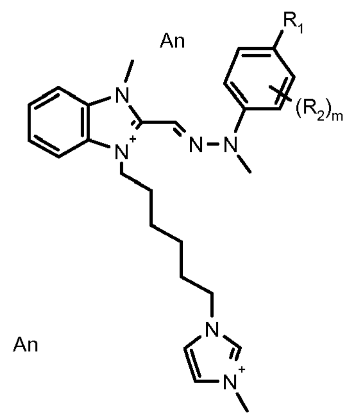

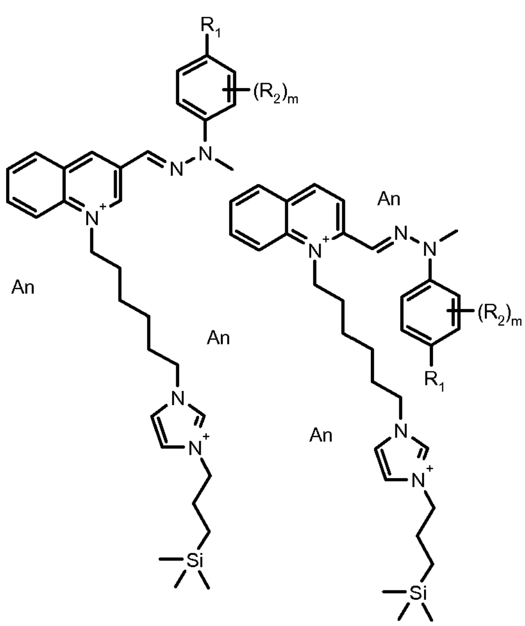

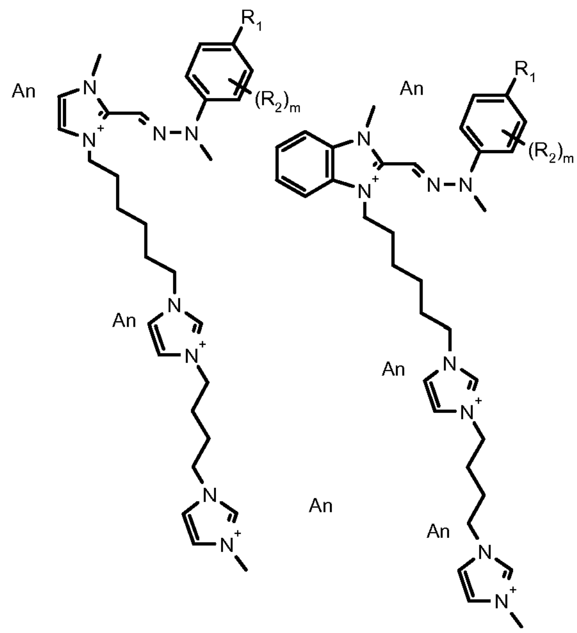

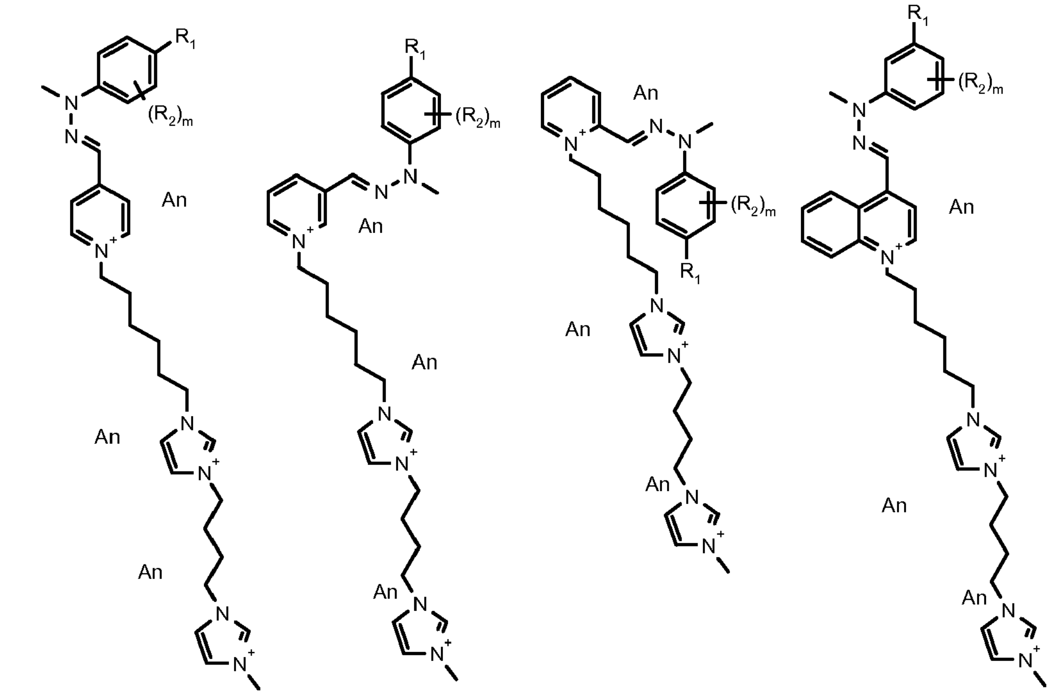

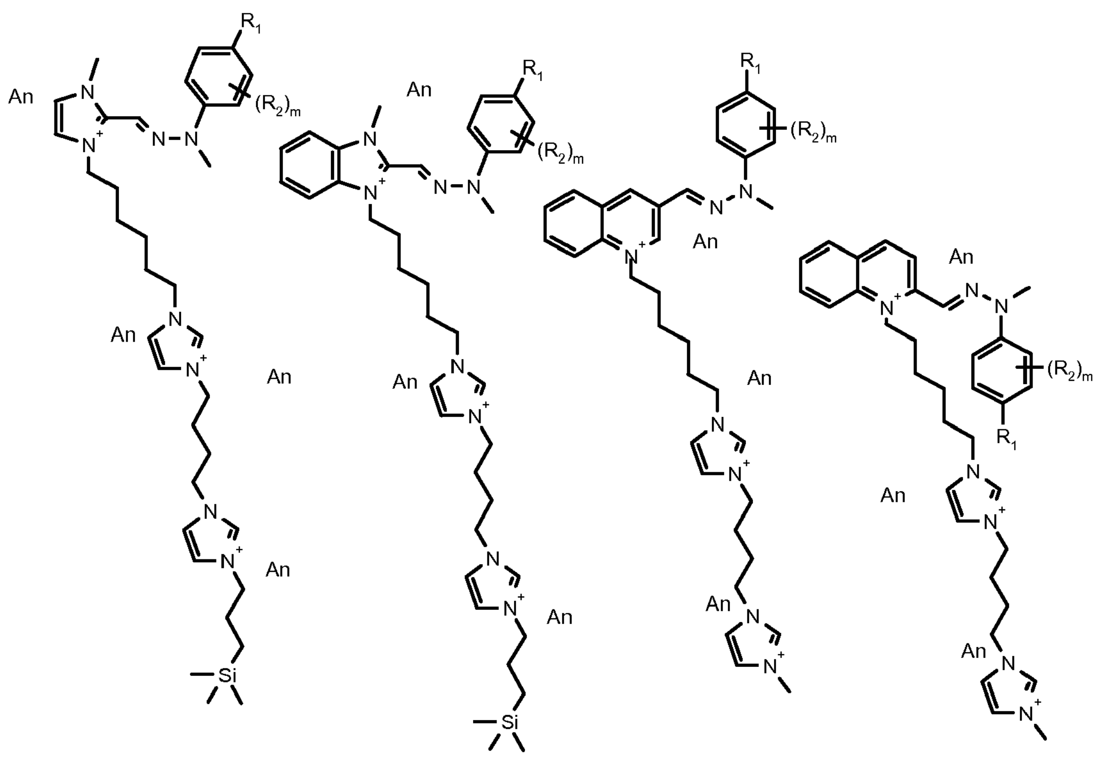

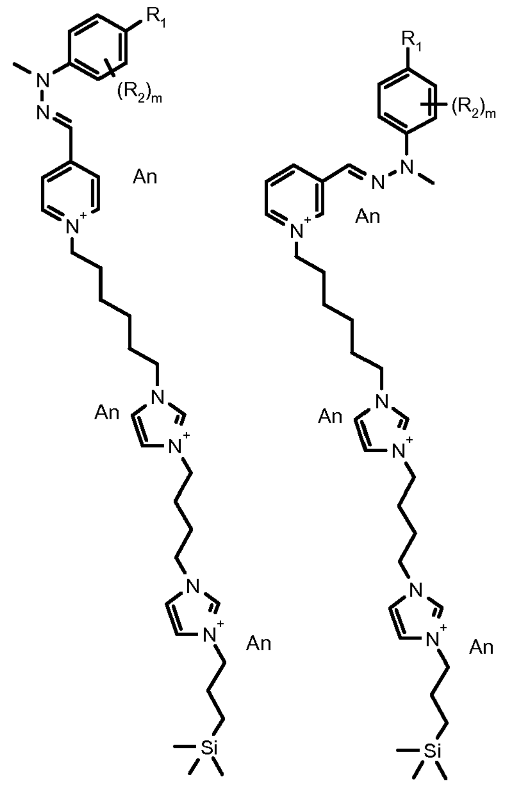

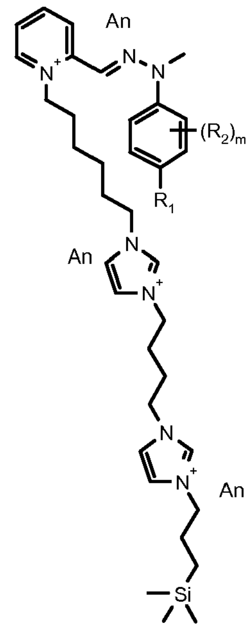

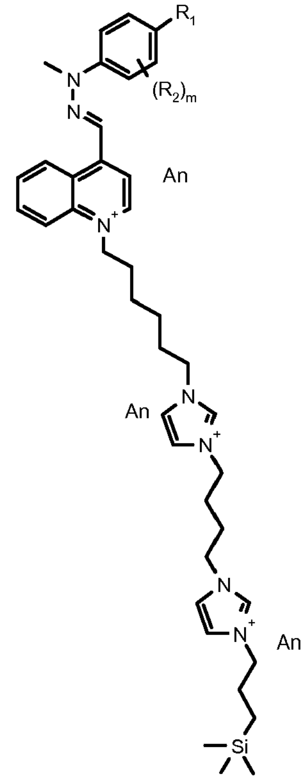

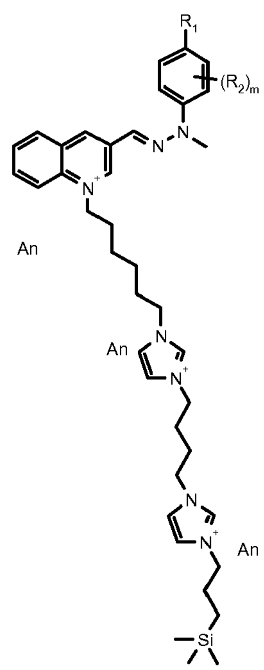

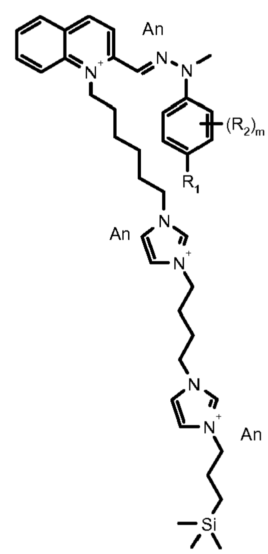

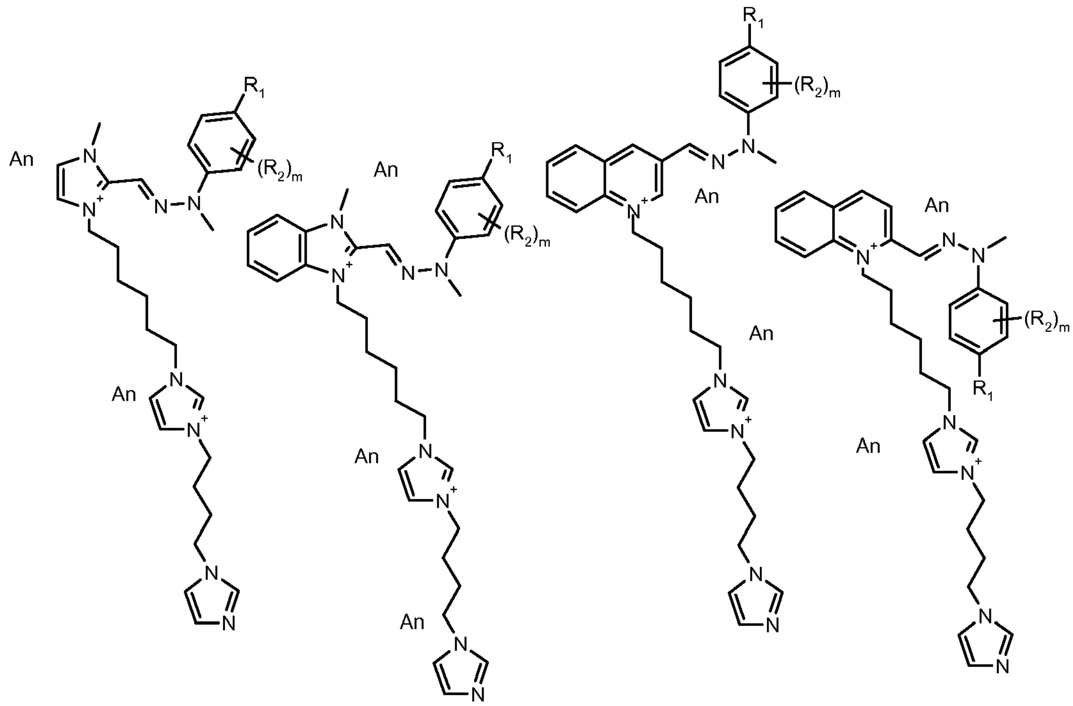

Formules dans lesquelles $R_1$, $R_2$, m, An ont les mêmes significations que celles indiquées précédemment, et R représente un atome d'hydrogène ou un radical méthyle.

**13.** Composition pour la coloration des fibres kératiniques humaines, comprenant dans un milieu cosmétiquement acceptable, un ou plusieurs composés polycationiques de formule (I) selon l'une quelconque des revendications 1 à 12.

**14.** Procédé de coloration de fibres kératiniques humaines, **caractérisé en ce que** l'on met en oeuvre les étapes suivantes :

- on applique la composition tinctoriale selon la revendication 13 sur les fibres kératiniques,
- on laisser pauser pendant un temps suffisant pour obtenir l'effet souhaité.

**15.** Dispositif à plusieurs compartiments, **caractérisé en ce qu'**il comprend un premier compartiment renfermant une composition telle que définie selon la revendication 13 et un second compartiment renfermant une composition comprenant au moins un agent oxydant.

Office européen des brevets

# RAPPORT DE RECHERCHE EUROPEENNE

Numéro de la demande
EP 08 15 4420

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 1 647 548 A (OREAL [FR]) 19 avril 2006 (2006-04-19) * le document en entier * ----- | 1-15 | INV. C07D401/06 C07D401/14 C07D403/14 C09B26/04 |
| X | EP 1 757 660 A (OREAL [FR]) 28 février 2007 (2007-02-28) * le document en entier * ----- | 1-15 | |
| A | US 2003/208856 A1 (MIYABE HAJIME [JP] ET AL) 13 novembre 2003 (2003-11-13) * le document en entier * ----- | 1-15 | |
| P,X | WO 2007/122340 A (OREAL [FR]; DAVID HERVE [FR]; GREAVES ANDREW [FR]; BARIL BERANGERE [FR]) 1 novembre 2007 (2007-11-01) * le document en entier * ----- | 1-15 | |

DOMAINES TECHNIQUES RECHERCHES (IPC)
C07D
C09B

Le présent rapport a été établi pour toutes les revendications

| Lieu de la recherche | Date d'achèvement de la recherche | Examinateur |
|---|---|---|
| Munich | 13 août 2008 | Gregoire, Ariane |

3

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 08 15 4420

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-08-2008

| Document brevet cité au rapport de recherche | Date de publication | Membre(s) de la famille de brevet(s) | Date de publication |
|---|---|---|---|
| EP 1647548 A | 19-04-2006 | BR PI0506035 A | 06-06-2006 |
| | | CN 1796371 A | 05-07-2006 |
| | | FR 2876690 A1 | 21-04-2006 |
| | | JP 2006348264 A | 28-12-2006 |
| | | KR 20060054013 A | 22-05-2006 |
| | | MX PA05011099 A | 20-04-2006 |
| EP 1757660 A | 28-02-2007 | FR 2889954 A1 | 02-03-2007 |
| | | JP 2007146122 A | 14-06-2007 |
| | | US 2007125261 A1 | 07-06-2007 |
| US 2003208856 A1 | 13-11-2003 | AUCUN | |
| WO 2007122340 A | 01-11-2007 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 9515144 A
- WO 9501772 A
- EP 714954 A
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988